# EUROPEAN PATENT APPLICATION

(11) **EP 4 257 583 A1**
(43) Date of publication of application: **11.10.2023**
(21) Application number: 21900778.8
(22) Date of filing: 30.09.2021
(51) Int. Cl.: C07D 207/06, A61K 31/402, A61K 31/4164, A61P 25/00, C07D 233/10

(54) **BIPHENYL PYRROLIDINE AND BIPHENYL DIHYDROIMIDAZOLE DERIVATIVES FOR INHIBITING ACTIVITY OF 5-HT7 SEROTONIN RECEPTOR, AND PHARMACEUTICAL COMPOSITION COMPRISING SAME AS ACTIVE INGREDIENT**

(30) Priority: 01.12.2020 KR 20200165497
(71) Applicant: Korea Institute of Science and Technology, Seoul 02792 (KR)
(72) Inventor: CHOO, Hyunah, Seoul 02792 (KR); JEON, Byungsun, Seoul 02792 (KR); KANG, Taek, Seoul 02792 (KR); KIM, Doyoung, Seoul 02792 (KR); LEE, Jieon, Seoul 02792 (KR)
(74) Representative: advotec.
(86) International application number: PCT/KR2021/013410
(87) International publication number: WO 2022/119090

(57) **Abstract**

The present disclosure relates to a compound represented by Structural Formula 1 or 2 or a pharmaceutically acceptable salt thereof, for inhibiting the activity of the 5-HT₇ serotonin receptor.

## Description

### [Technical Field]

The present disclosure relates to a pharmaceutical composition containing biphenylpyrrolidine and biphenyldihydroimidazole derivatives for inhibiting the activity of the 5-HT₇ serotonin receptor as an active ingredient, more particularly to a pharmaceutical composition containing biphenylpyrrolidine and biphenyldihydroimidazole derivatives, which are capable of inhibiting the activity of the 5-HT₇ serotonin receptor by acting as an antagonist in the β-arrestin signaling pathway, as an active ingredient.

### [Background Art]

The neurotransmitter serotonin triggers various physiological processes by acting on 14 different serotonin receptors distributed in various organs. Each receptor induces various physiological responses through interaction with serotonin. Among them, the 5-HT₇ receptor, which is the most recently found serotonin subtype receptor, is distributed a lot especially in the hypothalamus, thalamus, hippocampus, cortex, etc. and is known to play important roles such as body temperature regulation, biorhythms, learning and memory, sleep, hippocampal signaling, etc. In addition, it is known to be involved in neurological disorders such as depression, migraine, anxiety, developmental disorder, inflammatory pain, neuropathic pain, etc.

With the progress of researches on GPCRs, it has been known that there are signaling systems dependent on the activity of G protein and β-arrestin and the activity of the two signaling systems can be regulated depending on the structure of GPCR ligands. Therefore, various ligands are being developed. The 5-HT₇ receptor is one of GPCRs (G protein-coupled receptors) and an assay system capable of identifying the presence and activity of the G protein signaling system and the β-arrestin signaling system have been developed (Journal of Medicinal Chemistry, 2018, 61, 7218). It is known that SB-269970, which is well known as a 5-HT₇ receptor antagonist, acts as an antagonist against the G protein and β-arrestin signaling systems.

However, it is necessary to develop various β-arrestin antagonists because researches on the activity of β-arrestin are insufficient.

### [References of Related Art]

### [Patent Documents]

(Patent document 1) Korean Patent Registration No. 10-1779991.

### [Disclosure]

### [Technical Problem]

The present disclosure is directed to providing a biphenylpyrrolidine/dihydroimidazole derivative, which is capable of inhibiting the activity of the 5-HT₇ serotonin receptor by acting as an antagonist of the β-arrestin pathway for the 5-HT₇ serotonin receptor, or a pharmaceutically acceptable salt thereof.

The present disclosure is also directed to providing a pharmaceutical composition for preventing or treating a central nervous system disease such as sleep disorder, depression, migraine, anxiety, pain, inflammatory pain, neuropathic pain, thermoregulation disorder, biorhythm disorder, developmental disorder including autism spectrum disorder, smooth muscle disorder, etc., which contains the biphenylpyrrolidine/dihydroimidazole derivative or a pharmaceutically acceptable salt thereof as an active ingredient.

### [Technical Solution]

In an aspect, the present disclosure provides a compound for inhibiting the activity of the 5-HT₇ serotonin receptor, which is represented by Structural Formula 1 or 2, or a pharmaceutically acceptable salt thereof.

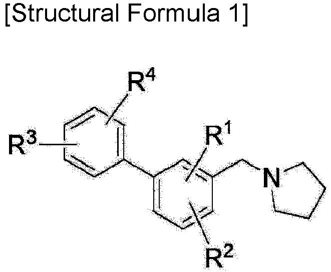

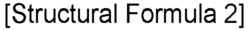

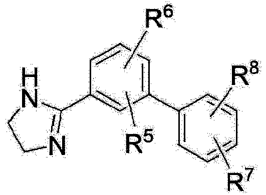

In Structural Formula 1 or 2,
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, the compound represented by Structural Formula 1 or 2 may be represented by Structural Formula 3 or 4.

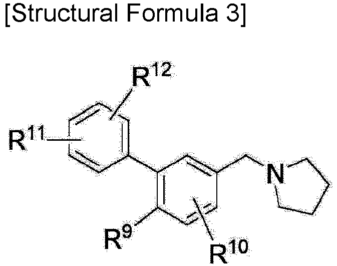

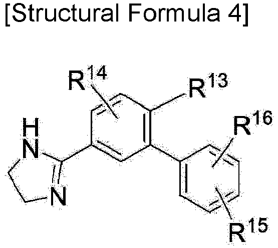

In Structural Formula 3 or 4,
each of R⁹ to R¹⁶, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, each of R⁹ to R¹⁶, which are identical to or different from each other, may be independently a hydrogen atom, a chloro group, a C₁-C₄ alkyl group or a C₁-C₄ alkoxy group.

The compound represented by Structural Formula 1 or 2 may be any one selected from the following compounds 1-44.

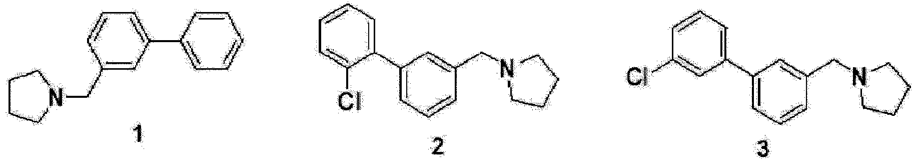

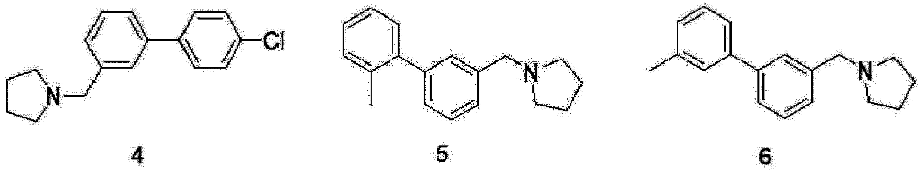

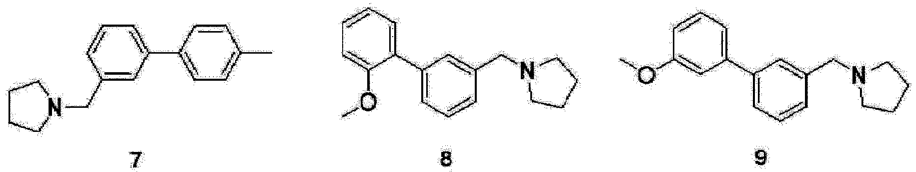

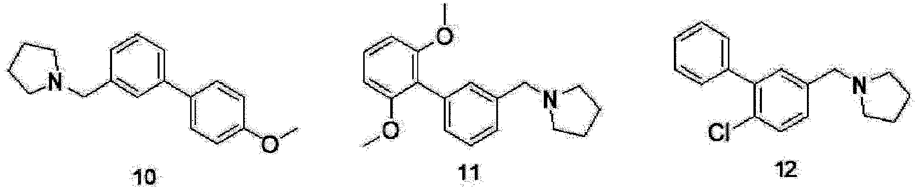

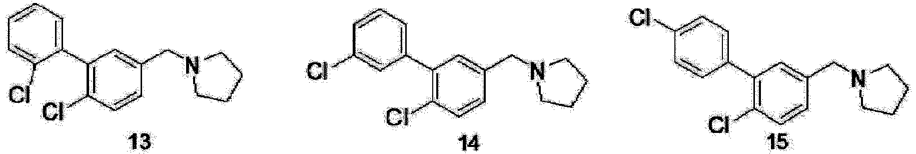

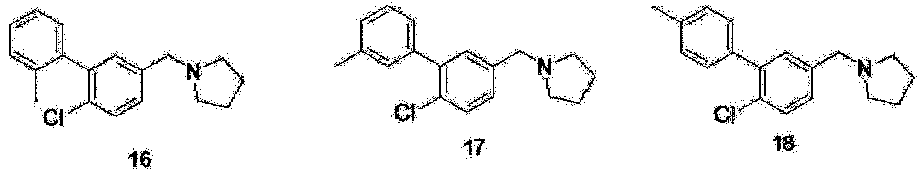

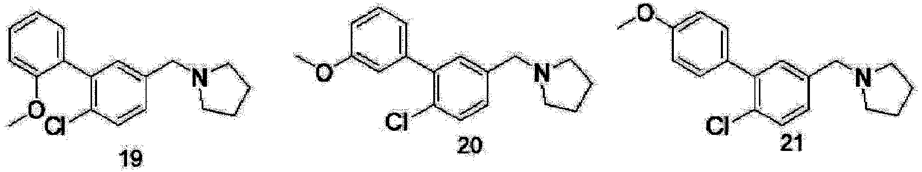

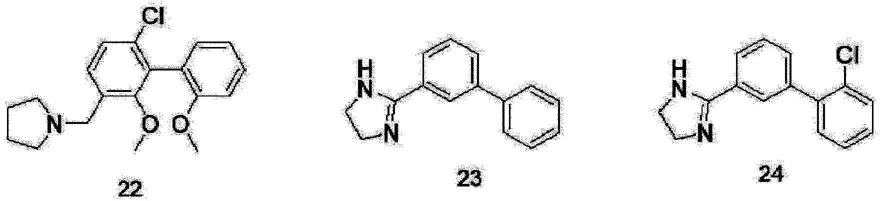

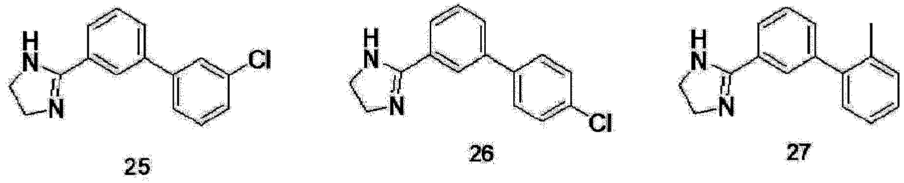

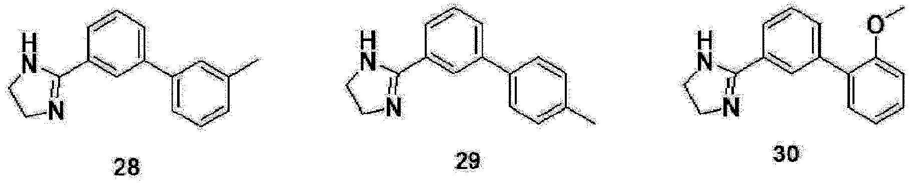

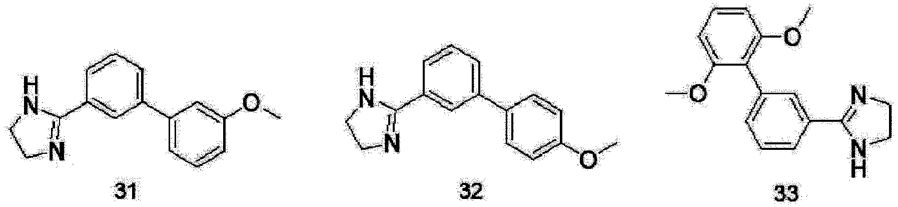

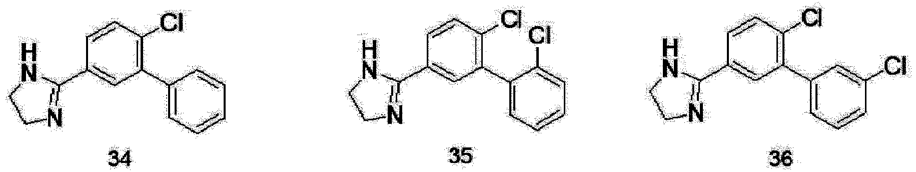

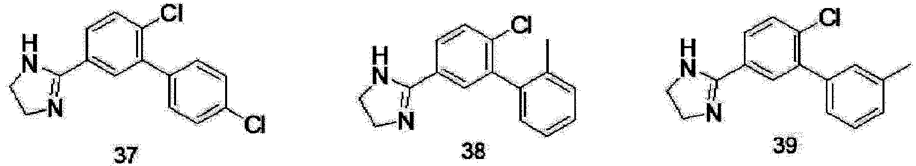

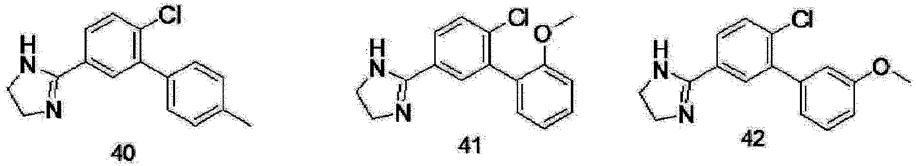

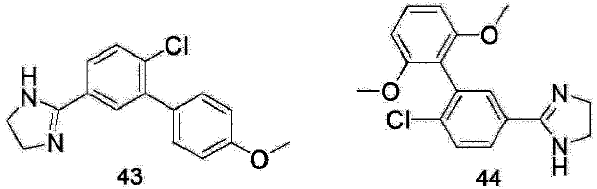

The pharmaceutically acceptable salt may be a salt formed using any inorganic acid or organic acid selected from hydrochloric acid, bromic acid, sulfonic acid, amidosulfuric acid, phosphoric acid, nitric acid, acetic acid, propionic acid, succinic acid, glycolic acid, stearic acid, lactic acid, tartaric acid, citric acid, p-toluenesulfonic acid and methanesulfonic acid.

In another aspect, the present disclosure provides a method for preparing a compound represented by Structural Formula 1 or 2 for inhibiting the activity of the 5-HT₇ serotonin receptor, which includes a step of reacting a compound represented by Structural Formula A with pyrrolidine or ethylenediamine.

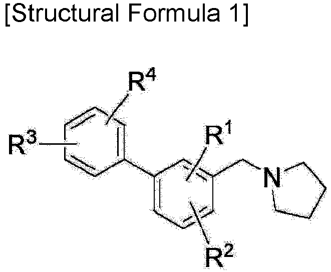

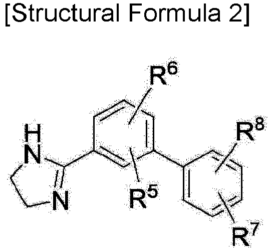

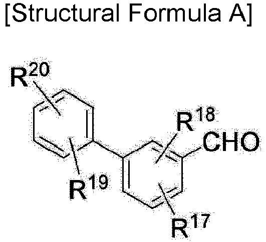

In Structural Formula 1 or 2,
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group, and

in Structural Formula B,
each of R¹⁷ to R²⁰, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, the compound represented by Structural Formula 1 or 2 may be represented by Structural Formula 3 or 4, and
the compound represented by Structural Formula A may be represented by Structural Formula B.

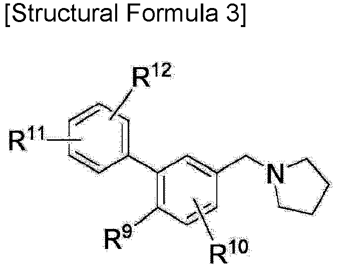

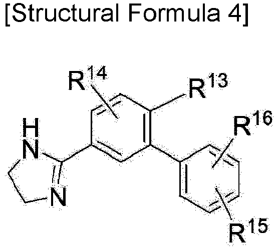

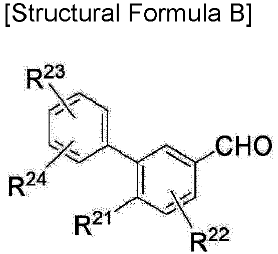

In Structural Formula 3 or 4,
each of R⁹ to R¹⁶, which are identical to or different from each other, is independently a hydrogen atom, a halogen group, a substituted or unsubstituted C₁-C₁₀ alkyl group or a substituted or unsubstituted C₁-C₁₀ alkoxy group, and

in Structural Formula B,
each of R²¹ to R²⁴, which are identical to or different from each other, is independently a hydrogen atom, a halogen group, a substituted or unsubstituted C₁-C₁₀ alkyl group or a substituted or unsubstituted C₁-C₁₀ alkoxy group.

The compound represented by Structural Formula B may be prepared by the Suzuki reaction of a compound represented by Structural Formula C and a compound represented by Structural Formula D.

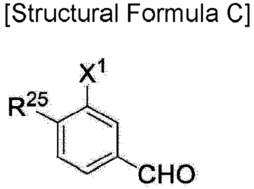

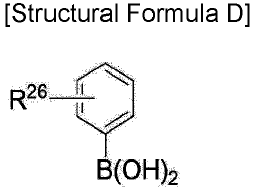

In Structural Formula C or D,
X₁ is a bromo group or an iodo group, and
each of R²⁵ and R²⁶, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, R²⁵ may be a hydrogen atom and X₁ may be a bromo group.

Also, specifically, R²⁶ may be a chloro group and X₁ may be an iodo group.

In another aspect, the present disclosure provides a pharmaceutical composition for preventing or treating a central nervous system disease, which contains a compound represented by Structural Formula 1 or 2 or a pharmaceutically acceptable salt thereof as an active ingredient.

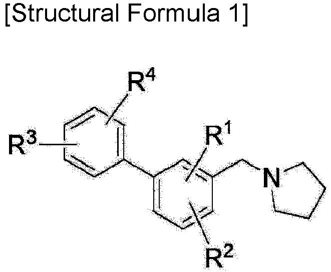

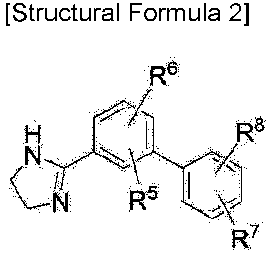

In Structural Formula 1 or 2,
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group.

Most specifically, the compound represented by Structural Formula 1 or 2 may be any one selected from the following compounds 1-44.

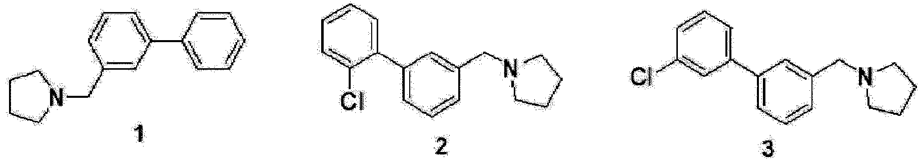

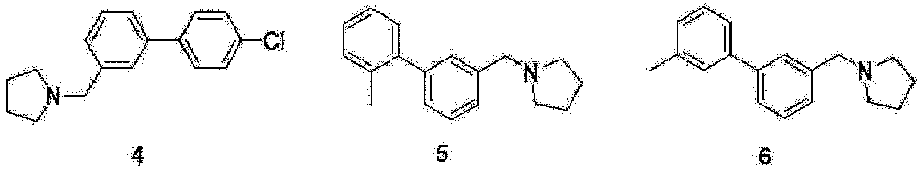

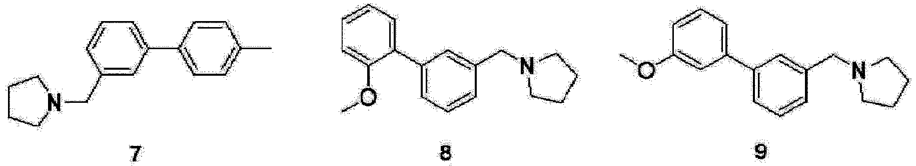

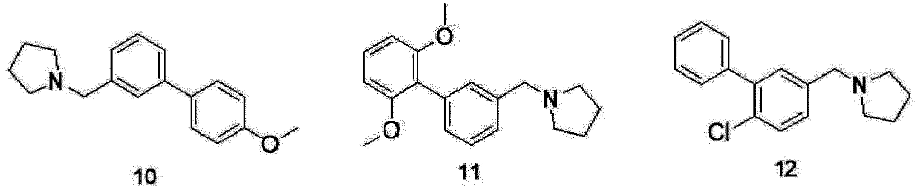

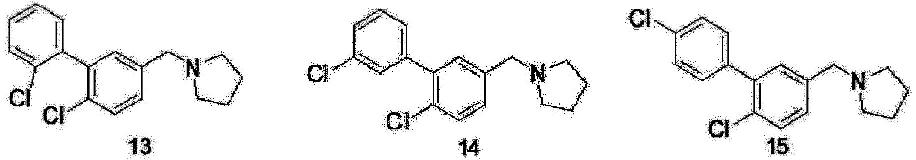

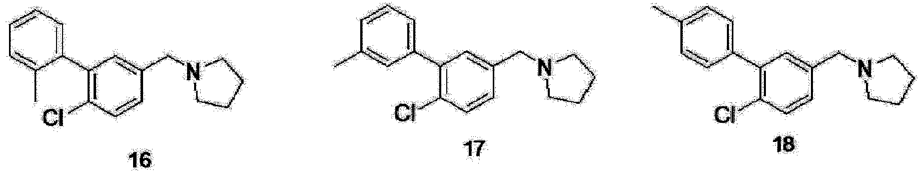

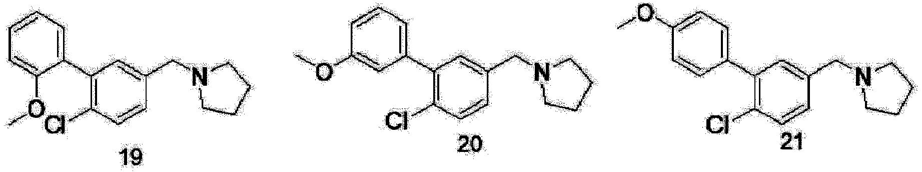

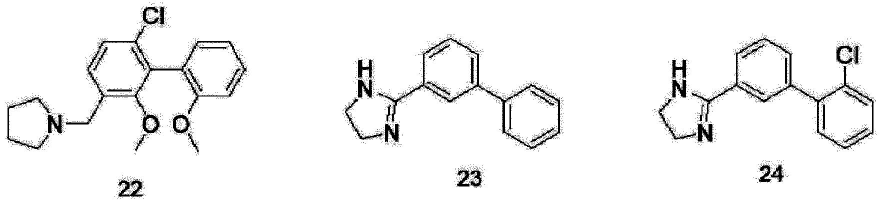

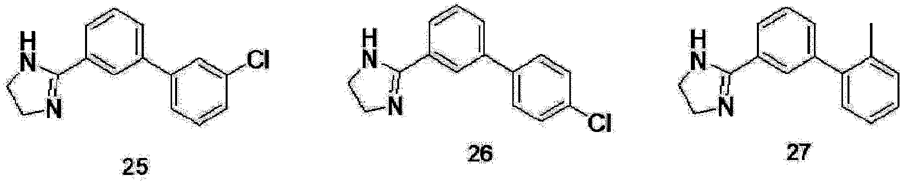

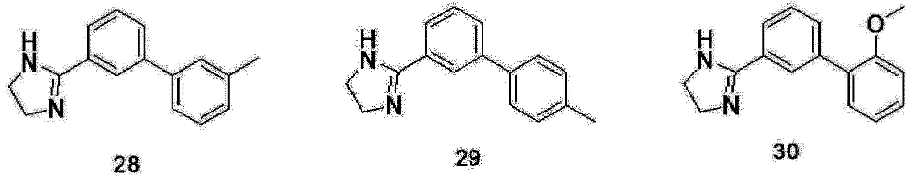

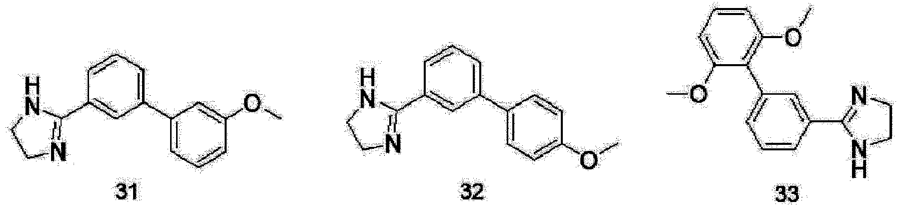

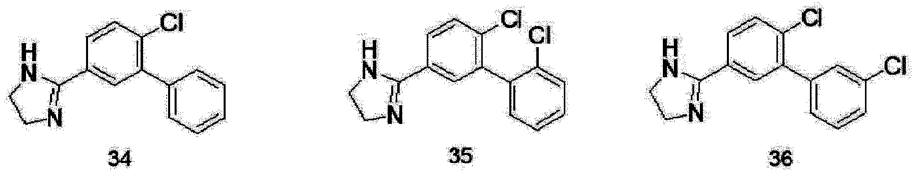

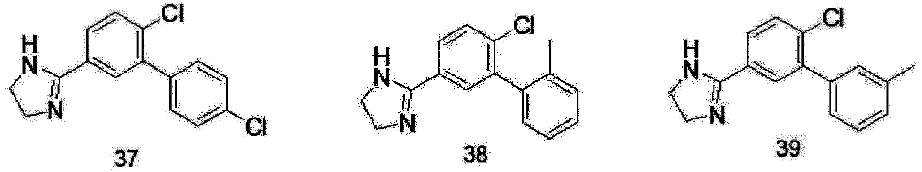

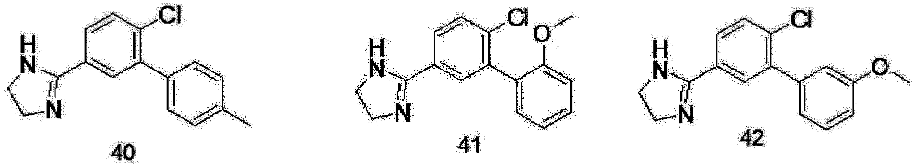

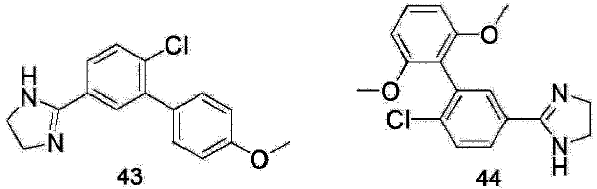

The central nervous system disease may be any disease selected from sleep disorder, depression, migraine, anxiety, pain, inflammatory pain, neuropathic pain, thermoregulation disorder, biorhythm disorder, autism spectrum disorder and smooth muscle disorder.

### [Advantageous Effects]

The biphenylpyrrolidine/dihydroimidazole derivative of the present disclosure and a pharmaceutically acceptable salt thereof can inhibit the activity of the 5-HT₇ serotonin receptor because it exhibits superior binding affinity and superior antagonistic activity for the 5-HT₇ serotonin receptor. Accordingly, a pharmaceutical composition containing the same as an active ingredient is effective in preventing or treating a brain disease including a central nervous system disease, specifically, depression, migraine, anxiety, pain, inflammatory pain, neuropathic pain, thermoregulation disorder, biorhythm disorder, sleep disorder, developmental disorder including autism spectrum disorder, smooth muscle-related disease, etc. that requires the inhibition of the activity of the 5-HT₇ serotonin receptor.

### [Brief Description of Drawings]

FIGS. 1A and 1B show results of Tango assay in Test Example 2.
FIG. 2 shows a result of measuring the pA₂ value using a Schild plot for Tango assay in Test Example 2.

### [Best Mode]

The present disclosure may be changed variously and may have various exemplary embodiments. Hereinafter, the specific exemplary embodiments of the present disclosure will be described in detail. However, the present disclosure is not limited to specific exemplary embodiments but encompasses all changes, equivalents and substitutes included within the technical idea and scope of the present disclosure. When describing the present disclosure, detailed description of well-known matters may be omitted to avoid unnecessarily obscuring the present disclosure.

The term "substituted" means that at least one hydrogen atom is substituted with a substituent selected from a group consisting of deuterium, a C₁-C₃₀ alkyl group, a C₃-C₃₀ cycloalkyl group, a C₂-C₃₀ heterocycloalkyl group, a C₁-C₃₀ haloalkyl group, a C₆-C₃₀ aryl group, a C₁-C₃₀ heteroaryl group, a C₁-C₃₀ alkoxy group, a C₃-C₃₀ cycloalkoxy group, a C₁-C₃₀ heterocycloalkoxy group, a C₂-C₃₀ alkenyl group, a C₂-C₃₀ alkynyl group, a C₆-C₃₀ aryloxy group, a C₁-C₃₀ heteroaryloxy group, a silyloxy group (-OSiHs), -OSiR¹H₂ (R¹ is a C₁-C₃₀ alkyl group or a C₆-C₃₀ aryl group), -OSiR¹R²H (each of R¹ and R² is independently a C₁-C₃₀ alkyl group or a C₆-C₃₀ aryl group),-OSiR¹R²R³ (each of R¹, R² and R³ is independently a C₁-C₃₀ alkyl group or a C₆-C₃₀ aryl group), a C₁-C₃₀ acyl group, a C₂-C₃₀ acyloxy group, a C₂-C₃₀ heteroaryloxy group, a C₁-C₃₀ sulfonyl group, a C₁-C₃₀ alkylthio group, a C₃-C₃₀ cycloalkylthio group, a C₁-C₃₀ heterocycloalkylthio group, a C₆-C₃₀ arylthio group, a C₁-C₃₀ heteroarylthio group, a C₁-C₃₀ phosphoramide group, a silyl group (SiR¹R²R³) (each of R¹, R² and R³ is independently a hydrogen atom, a C₁-C₃₀ alkyl group or a C₆-C₃₀ aryl group), an amine group (-NRR') (each of R and R' is independently a substituent selected from a group consisting of a hydrogen atom, a C₁-C₃₀ alkyl group and a C₆-C₃₀ aryl group), a carboxyl group, a halogen group, a cyano group, a nitro group, an azo group and a hydroxyl group.

In addition, two neighboring substituents may be fused to form a saturated or unsaturated ring.

The number of carbons in the alkyl group or the aryl group of "substituted or unsubstituted C₁-C₃₀ alkyl group", "substituted or unsubstituted C₆-C₃₀ aryl group", etc. means the number of the carbons constituting the alkyl or aryl moiety without considering the substituent. For example, a phenyl group in which a butyl group is substituted at the para-position is a C₆ aryl group substituted with a C₄ butyl group.

In the present specification, "hydrogen" refers to hydrogen, deuterium or tritium unless defined otherwise.

In the present specification, the term "alkyl group" refers to an aliphatic hydrocarbon group unless defined otherwise.

The alkyl group may be a "saturated alkyl group" containing no double or triple bond.

The alkyl group may also be an "unsaturated alkyl group" containing at least one double or triple bond.

The alkyl group may be branched, straight or cyclic whether it is saturated or unsaturated.

The alkyl group may be a C₁-C₃₀ alkyl group. More specifically, it may be a C₁-C₂₀ alkyl group, a C₁-C₁₀ alkyl group or a C₁-C₆ alkyl group.

For example, a C₁-C₄ alkyl group has 1-4 carbon atoms in the alkyl chain. That is to say, the C₁-C₄ alkyl group may be selected from a group consisting of methyl, ethyl, propyl, isopropyl, n-butyl, isobutyl, sec-butyl and t-butyl.

As specific examples, the alkyl group may be a methyl group, an ethyl group, a propyl group, an isopropyl group, a butyl group, an isobutyl group, a t-butyl group, a pentyl group, a hexyl group, an ethenyl group, a propenyl group, a butenyl group, a cyclopropyl group, a cyclobutyl group, a cyclopenyl group, a cyclohexyl group, etc.

In the present specification, the expression 'containing as an active ingredient' means that the 2,6-diarylenebenzoxazole derivative or a pharmaceutically acceptable salt thereof is contained in an amount sufficient to achieve the desired efficacy or activity. For example, the 2,6-diarylenebenzoxazole derivative or a pharmaceutically acceptable salt thereof may be used at a concentration of 10-1500 µg/mL, specifically 100-1000 µg/mL. However, the scope of the present disclosure is not limited thereto and the upper limit of the amount of the 2,6-diarylenebenzoxazole derivative contained in the pharmaceutical composition of the present disclosure may be selected adequately by those skilled in the art.

Hereinafter, the compound for inhibiting the activity of the 5-HT₇ serotonin receptor or a pharmaceutically acceptable salt thereof of the present disclosure will be described in detail.

The compound for inhibiting the activity of the 5-HT₇ serotonin receptor of the present disclosure is represented by Structural Formula 1 or 2.

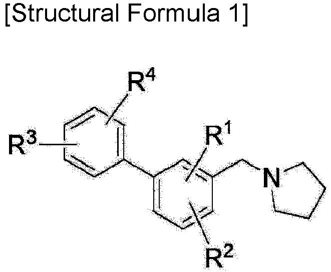

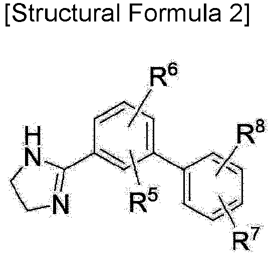

In Structural Formula 1 or 2,
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, the compound represented by Structural Formula 1 or 2 may be represented by Structural Formula 3 or 4.

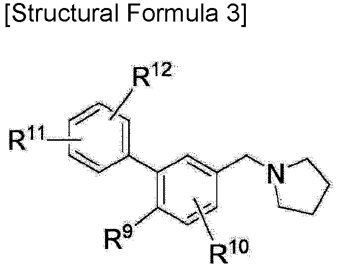

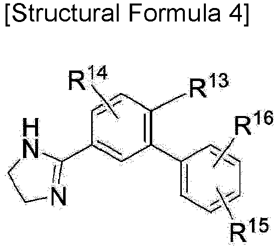

In Structural Formula 3 or 4,
each of R⁹ to R¹⁶, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, each of R⁹ to R¹⁶, which are identical to or different from each other, may independently be a hydrogen atom, a chloro group, a C₁-C₄ alkyl group or a C₁-C₄ alkoxy group.

Most specifically, the compound represented by Structural Formula 1 or 2 may be any one selected from the following compounds 1-44.

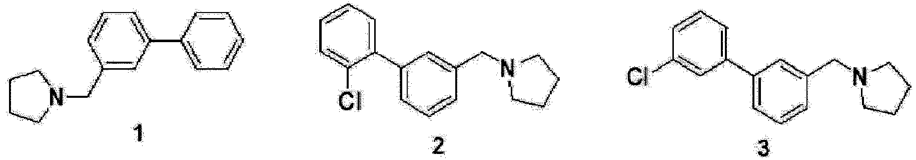

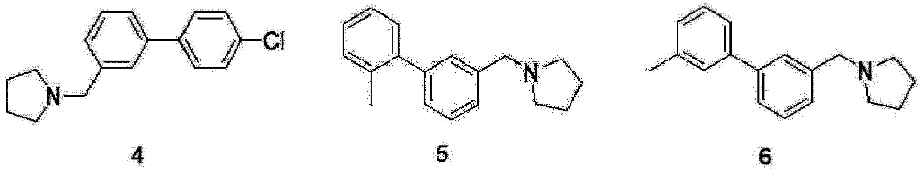

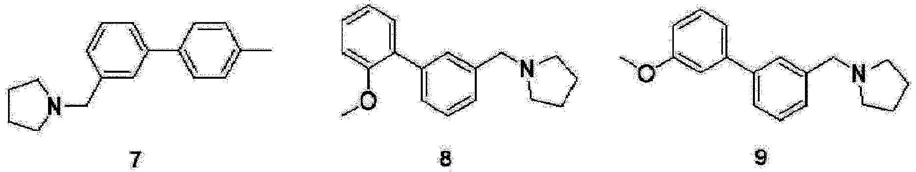

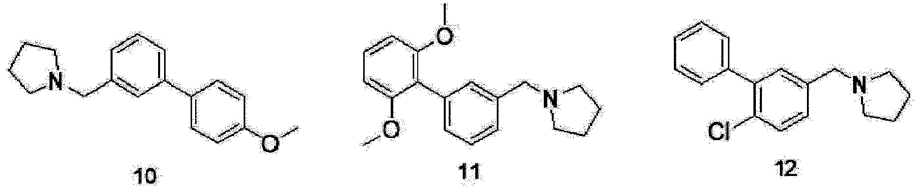

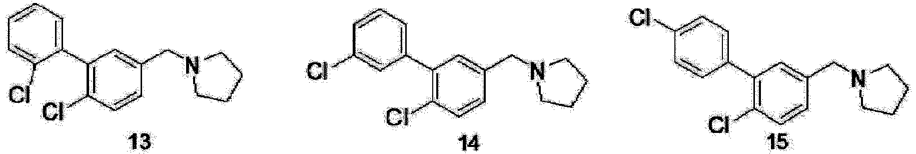

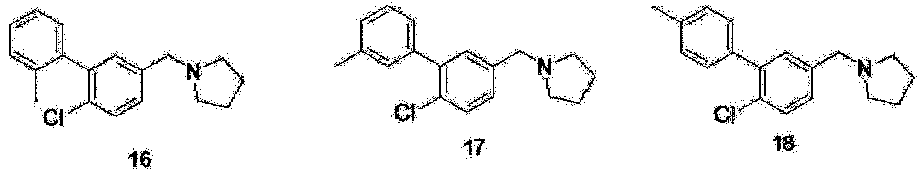

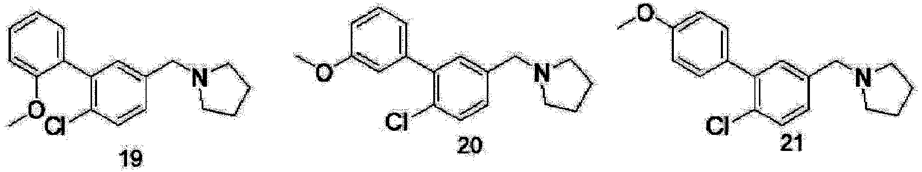

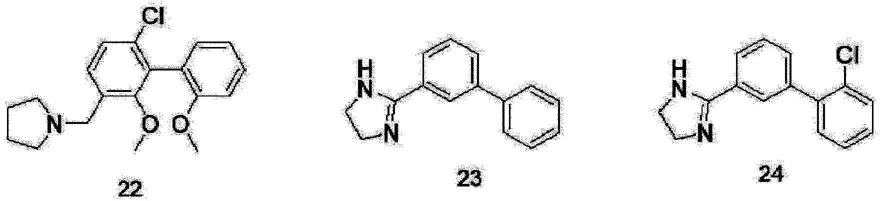

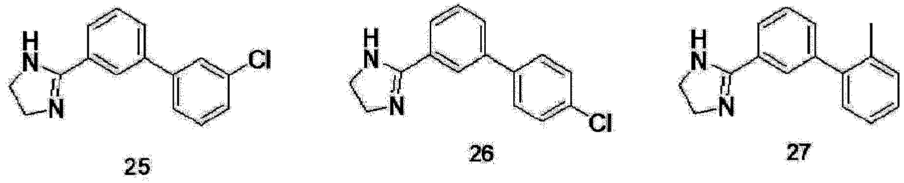

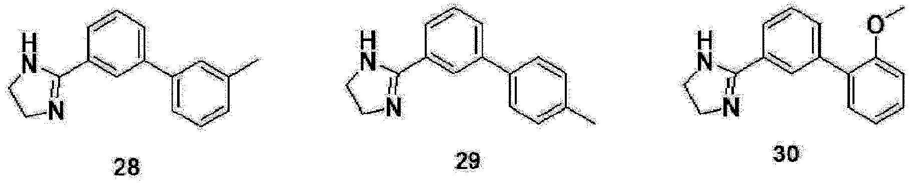

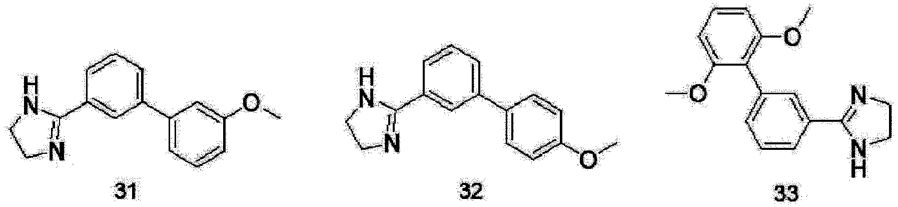

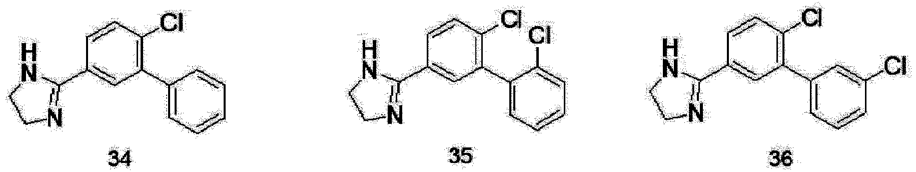

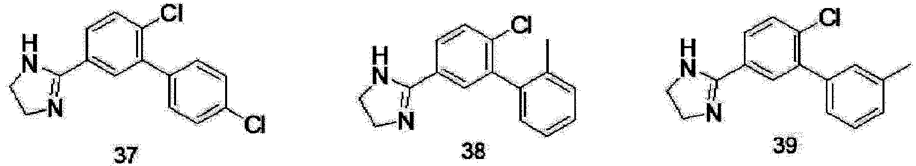

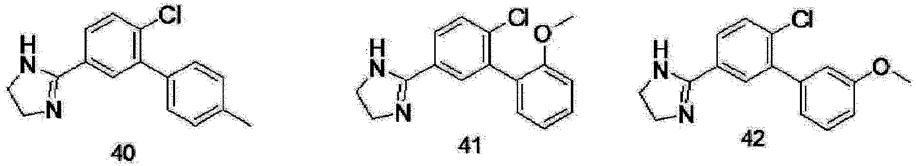

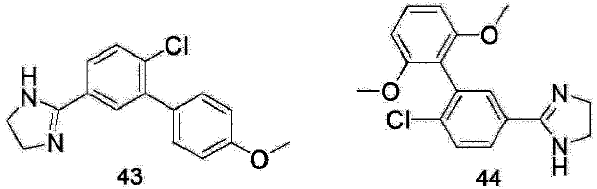

The pharmaceutically acceptable salt may be a salt formed using any inorganic acid or organic acid selected from hydrochloric acid, bromic acid, sulfonic acid, amidosulfuric acid, phosphoric acid, nitric acid, acetic acid, propionic acid, succinic acid, glycolic acid, stearic acid, lactic acid, tartaric acid, citric acid, p-toluenesulfonic acid and methanesulfonic acid.

Hereinafter, a method for preparing the compound for inhibiting the activity of the 5-HT₇ serotonin receptor or a pharmaceutically acceptable salt thereof according to the present disclosure will be described.

The method for preparing the compound for inhibiting the activity of the 5-HT₇ serotonin receptor or a pharmaceutically acceptable salt thereof according to the present disclosure may include a step of reacting a compound represented by Structural Formula A with pyrrolidine or ethylenediamine.

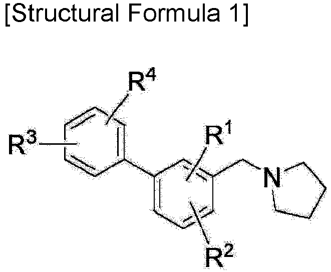

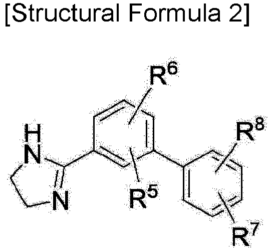

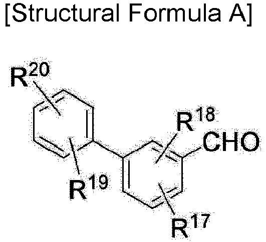

In Structural Formula 1 or 2,
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group, and,

in Structural Formula B,
each of R¹⁷ to R²⁰, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, the compound represented by Structural Formula 1 or 2 may be represented by Structural Formula 3 or 4, and the compound represented by Structural Formula A may be represented by Structural Formula B.

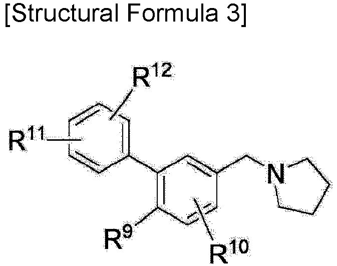

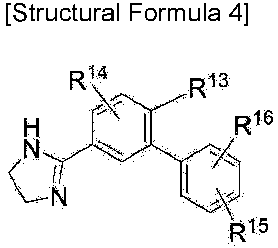

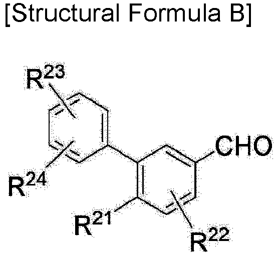

In Structural Formula 3 or 4,
each of R⁹ to R¹⁶, which are identical to or different from each other, is independently a hydrogen atom, a halogen group, a substituted or unsubstituted C₁-C₁₀ alkyl group or a substituted or unsubstituted C₁-C₁₀ alkoxy group, and,

in Structural Formula B,
each of R²¹ to R²⁴, which are identical to or different from each other, is independently a hydrogen atom, a halogen group, a substituted or unsubstituted C₁-C₁₀ alkyl group or a substituted or unsubstituted C₁-C₁₀ alkoxy group.

The compound represented by Structural Formula B may be prepared by the Suzuki reaction of a compound represented by Structural Formula C with a compound represented by Structural Formula D.

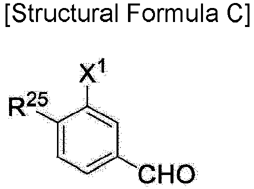

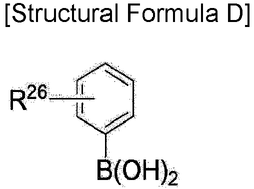

In Structural Formula C or D,
X₁ is a bromo group or an iodo group, and
each of R²⁵ and R²⁶, which are identical to or different from each other, is independently a hydrogen atom, a halogen group, a substituted or unsubstituted C₁-C₁₀ alkyl group or a substituted or unsubstituted C₁-C₁₀ alkoxy group.

Specifically, R²⁵ may be a hydrogen atom and X₁ may be a bromo group.

Also, specifically, R²⁶ may be a chloro group and X₁ may be an iodo group.

The present disclosure provides a pharmaceutical composition for preventing or treating a central nervous system disease, which contains a compound represented by Structural Formula 1 or 2 or a pharmaceutically acceptable salt thereof as an active ingredient.

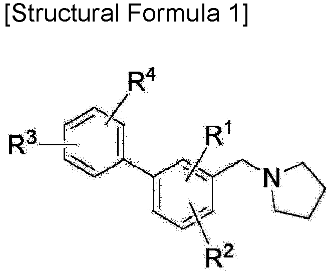

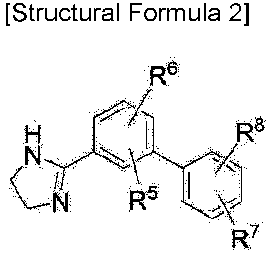

In Structural Formula 1 or 2,
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group.

The description about the compound represented by Structural Formula 1 or 2 will be omitted because it was described in detail above.

The central nervous system disease may be any disease selected from sleep disorder, depression, migraine, anxiety, pain, inflammatory pain, neuropathic pain, thermoregulation disorder, biorhythm disorder, autism spectrum disorder and smooth muscle disorder.

The pharmaceutical composition of the present disclosure may be prepared using a pharmaceutically acceptable and physiologically allowed adjuvant in addition to the active ingredient. The adjuvant may be an excipient, a disintegrant, a sweetener, a binder, a coating agent, a swelling agent, a lubricant, a glidant, a flavorant, etc.

The pharmaceutical composition may be prepared into a formulation using one or more pharmaceutically acceptable carrier in addition to the active ingredient described above.

The pharmaceutical composition may be prepared into a formulation such as a granule, a powder, a tablet, a coated tablet, a capsule, a suppository, a liquid, a syrup, a juice, a suspension, an emulsion, a medicinal drop, an injectable liquid, etc. For example, for formulation into a tablet or a capsule, the active ingredient may be bound to an oral, nontoxic, pharmaceutically acceptable inert carrier such as ethanol, glycerol, water, etc. Also, if desired or necessary, a suitable binder, lubricant, disintegrant or coloring agent may be added. The suitable binder includes but is not limited to starch, gelatin, natural sugar such as glucose or β-lactose, corn sweetener, natural or synthetic gum such as acacia, tragacanth or sodium oleate, sodium stearate, magnesium stearate, sodium benzoate, sodium acetate, sodium chloride, etc. The disintegrant includes but is not limited to starch, methyl cellulose, agar, bentonite, xanthan gum, etc.

For a composition formulated into a liquid solution, one or more of saline, sterile water, Ringer's solution, buffered saline, albumin injection solution, dextrose solution, maltodextrin solution, glycerol and ethanol may be added as an acceptable pharmaceutical carrier suitable for sterilization and biological use and, if necessary, another common additive such as an antioxidant, a buffer, a bacteriostat, etc. may be added. In addition, an injectable formulation such as an aqueous solution, a suspension, an emulsion, etc., a pill, a capsule, a granule or a tablet may be formulated by additionally adding a diluent, a dispersant, a surfactant, a binder or a lubricant.

The pharmaceutical composition of the present disclosure may be administered orally or parenterally. The parenteral administration may be accomplished by intravenous injection, subcutaneous injection, intramuscular injection, intraperitoneal injection, transdermal injection, etc. Specifically, it may be administered orally.

The adequate administration dosage of the pharmaceutical composition of the present disclosure varies depending on various factors such as formulation method, administration method, the age, body weight, sex, pathological condition and diet of a patient, administration time, administration route, excretion rate and response sensitivity. An ordinarily skilled physician can easily determine and prescribe an administration dosage effective for the desired treatment or prevention. According to a specific exemplary embodiment of the present disclosure, a daily administration dosage of the pharmaceutical composition of the present disclosure is 0.001-10 g/kg.

The pharmaceutical composition of the present disclosure may be prepared into a single-dose or multiple-dose formulation using a pharmaceutically acceptable carrier and/or excipient. The formulation may be in the form of a solution in an oily or aqueous medium, a suspension, an emulsion, an extract, a powder, a granule, a tablet or a capsule, and may further contain a dispersant or a stabilizer.

In addition, the present disclosure provides a use of the compound represented by Structural Formula 1 or 2 or a pharmaceutically acceptable salt thereof for preparation of a drug for treating a central nervous system disease.

In addition, the present disclosure relates to a method for treating a central nervous system disease, which includes administering the compound represented by Structural Formula 1 or 2 or a pharmaceutically acceptable salt thereof to a mammal.

### [Examples]

### Example 1. Preparation of compound 1

### Step 1: Preparation of [1,1'-biphenyl]-3-carbaldehyde

After dissolving 3-bromobenzaldehyde (1.0 mmol), phenylboronic acid (1.2 mmol), PdCl₂(PPh₃)₂ (0.1 mmol) and Na₂CO₃ (4.0 mmol) in tetrahydrofuran (10 mL) in a reaction vessel, the reaction mixture was refluxed for 24 hours while heating at 70 °C. After cooling to room temperature and adding distilled water, the reaction mixture was extracted with dichloromethane. The obtained organic layer was dried with anhydrous magnesium sulfate and then filtered. The filtrate was concentrated under reduced pressure and the obtained concentrate was separated by column chromatography (hexane:ethyl acetate = 20:1) to obtain the target compound (yield: 91%).

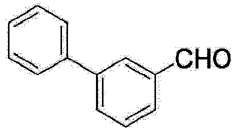

¹H NMR (400 MHz, CDCl₃) δ 10.12 (s, 1H), 8.13 (t, *J =* 1.8 Hz, 1H), 7.89 (dd, *J* = 7.7, 1.8 Hz, 2H), 7.68-7.61 (m, 3H), 7.54-7.48 (m, 2H), 7.46-7.41 (m, 1H).

¹³C NMR (100 MHz, CDCl₃) δ 192.35, 142.19, 139.72, 136.95, 133.08, 129.52, 129.03, 128.65, 128.22, 128.04, 127.17.

### Step 2: Preparation of 1-([1,1'-biphenyl]-3-ylmethyl)pyrrolidine

After adding the [1,1'-biphenyl]-3-carbaldehyde prepared in the step 1 (1.0 mmol) and pyrrolidine (2.0 mmol) in a reaction vessel and then dissolving with methanol, acetic acid (1.0 mmol) was added and the reaction mixture was stirred at room temperature for 2 hours. After adding sodium triacetoxyborohydride (3.0 mmol), the reaction mixture was stirred for 24 hours. After adding a saturated sodium bicarbonate solution to the reaction mixture and extracting with dichloromethane, the obtained organic layer was dried with anhydrous magnesium sulfate and then filtered. After concentrating the filtrate under reduced pressure, the obtained concentrate was separated by column chromatography (hexane:ethyl acetate = 3:1) to obtain compound 1 (yield: 36%).

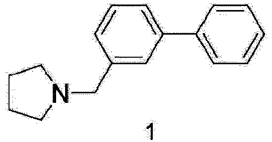

¹H NMR (400 MHz, CDCl₃) δ 7.67-7.61 (m, 2H), 7.61-7.58 (m, 1H), 7.53-7.49 (m, 1H), 7.49-7.39 (m, 3H), 7.39-7.32 (m, 2H), 3.72 (s, 2H), 2.61-2.51 (m, 4H), 1.85-1.79 (m, 4H).

¹³C NMR (100 MHz, CDCl₃) δ 141.50, 140.80, 136.50, 128.99, 128.78, 128.47, 128.43, 127.42, 127.20, 126.65, 59.54, 53.30, 23.28.

### Example 2: Preparation of 1-((2'-chloro-[1,1'-biphenyl]-3-yl)methyl)pyrrolidine

Compound 2 (yield: 86%) was obtained using the compound 2'-chloro-[1,1'-biphenyl]-3-carbaldehyde (yield: 85%) prepared in the same manner as in Example 1 using 3-bromobenzaldehyde (1.0 mmol), 2-chlorophenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

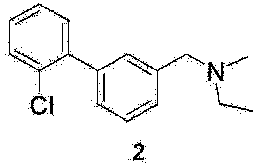

¹H NMR (400 MHz, CDCl₃) δ 7.42-7.31 (m, 2H), 7.34-7.27 (m, 2H), 7.30-7.23 (m, 2H), 7.21 (dd, *J* = 7.1, 1.9 Hz, 2H), 3.62 (s, 2H), 2.52-2.46 (m, 4H), 1.77-1.69 (m, 4H).

¹³C NMR (100 MHz, CDCl₃) δ 140.59, 139.32, 139.17, 132.55, 131.45, 130.00, 129.91, 128.45, 128.20, 127.98, 127.94, 126.76, 60.63, 54.19, 23.50.

### Example 3: Preparation of 1-((3'-chloro-[1,1'-biphenyl]-3-yl)methyl)pyrrolidine

Compound 3 (yield: 85%) was obtained using the compound 3'-chloro-[1,1'-biphenyl]-3-carbaldehyde (yield: 86%) prepared in the same manner as in Example 1 using 3-bromobenzaldehyde (1.0 mmol), 3-chlorophenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

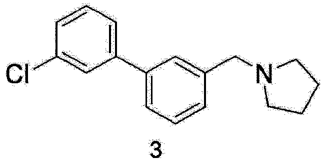

¹H NMR (400 MHz, CDCl₃) δ 7.63 (t, *J* = 1.9 Hz, 1H), 7.57 (t, *J* = 1.8 Hz, 1H), 7.51 (dt, *J* = 7.5, 1.6 Hz, 1H), 7.48 (dt, *J* = 7.5, 1.7 Hz, 1H), 7.44-7.35 (m, 3H), 7.35-7.31 (m, 1H), 3.71 (s, 2H), 2.60-2.54 (m, 4H), 1.87-1.80 (m, 4H).

¹³C NMR (100 MHz, CDCl₃) δ 143.11, 140.19, 139.77, 134.60, 129.93, 128.80, 128.48, 127.60, 127.35, 127.21, 125.69, 125.39, 60.74, 54.26, 23.51.

### Example 4: Preparation of 1-((4'-chloro-[1,1'-biphenyl]-3-yl)methyl)pyrrolidine

Compound 4 (yield: 77%) was obtained using the compound 4'-chloro-[1,1'-biphenyl]-3-carbaldehyde (yield: 78%) prepared in the same manner as in Example 1 using 3-bromobenzaldehyde (1.0 mmol), 4-chlorophenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

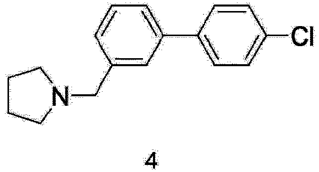

¹H NMR (400 MHz, CDCl₃) δ 7.59-7.53 (m, 3H), 7.47 (dt, *J* = 7.6, 1.7 Hz, 1H), 7.45-7.39 (m, 3H), 7.36 (dt, *J* = 7.4, 1.7 Hz, 1H), 3.71 (s, 2H), 2.62-2.53 (m, 4H), 1.87-1.79 (m, 4H).

¹³C NMR (100 MHz, CDCl₃) δ 140.12, 139.95, 139.69, 133.30, 128.84, 128.78, 128.47, 128.20, 127.49, 125.57, 60.74, 54.25, 23.50.

### Example 5: Preparation of 1-((2'-methyl-[1,1'-biphenyl]-3-yl)methyl)pyrrolidine

Compound 5 (yield: 68%) was obtained using the compound 2'-methyl-[1,1'-biphenyl]-3-carbaldehyde (yield: 93%) prepared in the same manner as in Example 1 using 3-bromobenzaldehyde (1.0 mmol), 2-methylphenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

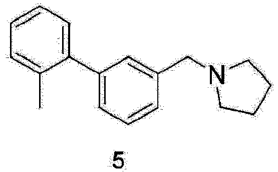

¹H NMR (400 MHz, CDCl₃) δ 7.43-7.32 (m, 2H), 7.36-7.25 (m, 4H), 7.29-7.21 (m, 2H), 3.70 (s, 2H), 2.62-2.50 (m, 4H), 2.31 (s, 3H), 1.89-1.75 (m, 4H).

¹³C NMR (100 MHz, CDCl₃) δ 141.98, 141.84, 139.17, 135.35, 130.26, 129.83, 129.77, 127.96, 127.73, 127.40, 127.18, 125.70, 60.72, 54.19, 23.49, 20.53.

### Example 6: Preparation of 1-((3'-methyl-[1,1'-biphenyl]-3-yl)methyl)pyrrolidine

Compound 6 (yield: 83%) was obtained using the compound 3'-methyl-[1,1'-biphenyl]-3-carbaldehyde (yield: 95%) prepared in the same manner as in Example 1 using 3-bromobenzaldehyde (1.0 mmol), 3-methylphenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

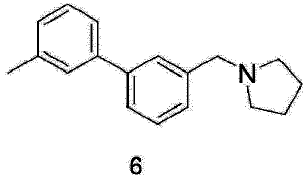

¹H NMR (400 MHz, CDCl₃) δ 7.59 (t, *J* = 1.9 Hz, 1H), 7.54-7.49 (dt, *J* = 7.5, 1.7 Hz, 1H), 7.47-7.39 (m, 3H), 7.38-7.33 (m, 2H), 7.19 (d, *J* = 7.5 Hz, 1H), 3.74 (s, 2H), 2.64-2.67 (m, 4H), 2.45 (s, 3H), 1.89-1.80 (m, 4H).

¹³C NMR (100 MHz, CDCl₃) δ 141.32, 141.19, 138.28, 128.65, 128.62, 128.03, 127.98, 127.91, 127.81, 125.86, 124.35, 60.72, 54.16, 23.46, 21.56.

### Example 7: Preparation of 1-((4'-methyl-[1,1'-biphenyl]-3-yl)methyl)pyrrolidine

Compound 7 (yield: 52%) was obtained using the compound 4'-methyl-[1,1'-biphenyl]-3-carbaldehyde (yield: 96%) prepared in the same manner as in Example 1 using 3-bromobenzaldehyde (1.0 mmol), 4-methylphenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

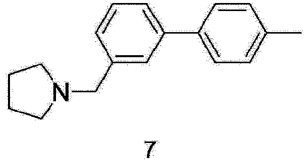

¹H NMR (400 MHz, CDCl₃) δ 7.59 (t, *J* = 1.8 Hz, 1H), 7.57-7.52 (m, 2H), 7.50 (dt, *J* = 7.6, 1.6 Hz, 1H), 7.40 (t, *J* = 7.6 Hz, 1H), 7.34 (dt, *J* = 7.6, 1.6 Hz, 1H), 7.27 (d, *J* = 8.2 Hz, 2H), 3.73 (s, 2H), 2.63-2.56 (m, 4H), 2.43 (s, 3H), 1.89-1.79 (m, 4H).

¹³C NMR (100 MHz, CDCl₃) δ 141.11, 139.67, 138.36, 136.95, 129.42, 128.63, 127.65, 127.56, 127.07, 125.59, 60.76, 54.18, 23.50, 21.11.

### Example 8: Preparation of 1-((2'-methoxy-[1,1'-biphenyl]-3-yl)methyl)pyrrolidine

Compound 8 (yield: 90%) was obtained using the compound 2'-methoxy-[1,1'-biphenyl]-3-carbaldehyde (yield: 92%) prepared in the same manner as in Example 1 using 3-bromobenzaldehyde (1.0 mmol), 2-methoxyphenylboronic acid (1.2 mmol), Pd(PPh₃)₄ (0.1 mmol) and Na₂CO₃ (4.0 mmol), pyrrolidine (2.0 mmol), acetic acid (1.0 mmol) and sodium triacetoxyborohydride (3.0 mmol).

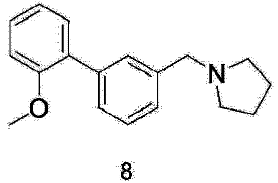

¹H NMR (400 MHz, CDCl₃) δ 7.52 (t, *J* = 1.8 Hz, 1H), 7.49-7.44 (dt, *J* = 7.3, 1.7 Hz, 1H), 7.42-7.31 (m, 4H), 7.08-7.02 (td, *J* = 7.5, 1.1 Hz, 1H), 7.02-6.98 (dd, *J* = 8.2, 1.1 Hz, 1H), 3.83 (s, 3H), 3.75 (s, 2H), 2.71-2.55 (m, 4H), 1.90-1.80 (m, 4H).

¹³C NMR (100 MHz, CDCl₃) δ 156.49, 138.46, 138.25, 130.96, 130.67, 130.24, 128.59, 128.30, 127.93, 127.68, 120.82, 111.24, 60.56, 55.58, 54.02, 23.46.

### Example 9: Preparation of 1-((3'-methoxy-[1,1'-biphenyl]-3-yl)methyl)pyrrolidine

Compound 9 (yield: 78%) was obtained using the compound 3'-methoxy-[1,1'-biphenyl]-3-carbaldehyde (yield: 96%) prepared in the same manner as in Example 1 using 3-bromobenzaldehyde (1.0 mmol), 3-methoxyphenylboronic acid (1.2 mmol), Pd(PPh₃)₄ (0.1 mmol) and Na₂CO₃ (4.0 mmol), pyrrolidine (2.0 mmol), acetic acid (1.0 mmol) and sodium triacetoxyborohydride (3.0 mmol).

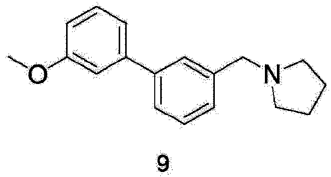

¹H NMR (400 MHz, CDCl₃) δ 7.48 (t, *J* = 1.7 Hz, 1H), 7.42-7.37 (dt, *J* = 7.5, 1.7 Hz, 1H), 7.33-7.22 (m, 3H), 7.14-7.09 (dt, *J* = 7.7, 1.3 Hz, 1H), 7.08-7.04 (m, 1H), 6.84-6.78 (ddd, *J* = 8.2, 2.6, 0.9 Hz, 1H), 3.78 (s, 3H), 3.61 (s, 2H), 2.48 (m, 4H), 1.76-1.68 (m, 4H).

¹³C NMR (100 MHz, CDCl₃) δ 159.93, 142.79, 141.06, 139.74, 129.69, 128.66, 128.09, 127.78, 125.84, 119.80, 112.99, 112.65, 60.74, 55.35, 54.21, 23.50.

### Example 10: Preparation of 1-((4'-methoxy-[1,1'-biphenyl]-3-yl)methyl)pyrrolidine

Compound 10 (yield: 95%) was obtained using the compound 4'-methoxy-[1,1'-biphenyl]-3-carbaldehyde (yield: 91%) prepared in the same manner as in Example 1 using 3-bromobenzaldehyde (1.0 mmol), 4-methoxyphenylboronic acid (1.2 mmol), Pd(PPh₃)₄ (0.1 mmol) and Na₂CO₃ (4.0 mmol), pyrrolidine (2.0 mmol), acetic acid (1.0 mmol) and sodium triacetoxyborohydride (3.0 mmol).

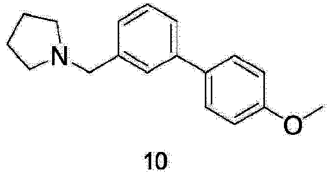

¹H NMR (400 MHz, CDCl₃) δ 7.61-7.55 (m, 3H), 7.50-7.45 (m, 1H), 7.40 (t, *J* = 7.5 Hz, 1H), 7.34-7.29 (m, 1H), 7.03-6.98 (m, 2H), 3.88 (s, 3H), 3.72 (s, 2H), 2.63-2.56 (m, 4H), 1.88-1.80 (m, 4H).

¹³C NMR (100 MHz, CDCl₃) δ 159.13, 140.78, 139.64, 133.77, 128.65, 128.24, 127.35, 127.32, 125.37, 114.15, 60.80, 55.36, 54.22, 23.50.

### Example 11: Preparation of 1-((2',6'-dimethoxy-[1,1'-biphenyl]-3-yl)methyl)pyrrolidine

Compound 11 (yield: 66%) was obtained using the compound 2',6'-dimethoxy-[1,1'-biphenyl]-3-carbaldehyde (yield: 89%) prepared in the same manner as in Example 1 using 3-bromobenzaldehyde (1.0 mmol), 2,6-dimethoxyphenylboronic acid (1.2 mmol), Pd(PPh₃)₄ (0.1 mmol) and Na₂CO₃ (4.0 mmol), pyrrolidine (2.0 mmol), acetic acid (1.0 mmol) and sodium triacetoxyborohydride (3.0 mmol).

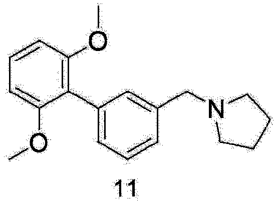

¹H NMR (400 MHz, CDCl₃) δ 7.38-7.33 (m, 1H), 7.31-7.27 (m, 2H), 7.27-7.22 (m, 2H), 6.64 (d, *J* = 8.4 Hz, 2H), 3.71 (s, 6H), 3.70 (s, 2H), 2.61-2.56 (m, 4H), 1.82-1.77 (m, 4H).

¹³C NMR (100 MHz, CDCl₃) δ 157.70, 137.63, 133.93, 131.80, 129.61, 128.59, 127.60, 127.55, 119.59, 104.31, 60.49, 55.93, 53.88, 23.47.

### Example 12: Preparation of 1-((6-chloro-[1,1'-bipheyl]-3-yl)methyl)pyrrolidine

### Step 1: Preparation of 4-chloro-3-iodobenzaldehyde

After dissolving iodine (0.44 mmol) and sodium iodate (0.22 mmol) in sulfuric acid (10 mL) in a reaction vessel, the reaction mixture was stirred for 30 minutes. After adding 4-chlorobenzaldehyde, the reaction mixture was stirred at room temperature for 2 hours. After cooling to 0 °C and adding distilled water to the reaction mixture, the produced solid was filtered. The filtered solid was added to a sodium thiosulfate solution and extracted with dichloromethane. The obtained organic layer was dried with anhydrous magnesium sulfate and then filtered. After concentrating the filtrate under reduced pressure, the obtained concentrate was separated by column chromatography (hexane:ethyl acetate = 10:1) to obtain the target compound (yield: 73%).

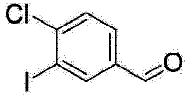

¹H NMR (400 MHz, CDCl₃) δ 9.85 (s, 1H), 8.28 (d, *J* = 1.9 Hz, 1H), 7.73 (dd, *J* = 8.2, 1.9 Hz, 1H), 7.55 (d, *J* = 8.2 Hz, 1H).

¹³C NMR (100 MHz, CDCl₃) δ 189.41, 145.07, 141.38, 135.70, 130.01, 129.96, 98.84.

### Step 2: Preparation of 6-chloro-[1,1'-biphenyl]3-carbaldehyde

After dissolving 4-chloro-3-iodobenzaldehyde (1.0 mmol), phenylboronic acid (1.2 mmol), PdCl₂(PPh₃)₂ (0.1 mmol) and Na₂CO₃ (4.0 mmol) in tetrahydrofuran (10 mL) in a reaction vessel, the reaction mixture was refluxed by heating at 70 °C for 24 hours. After cooling to room temperature and adding distilled water, the reaction mixture was extracted with dichloromethane. The obtained organic layer was dried with anhydrous magnesium sulfate and then filtered. After concentrating the filtrate under reduced pressure, the obtained concentrate was separated by column chromatography (hexane:ethyl acetate = 20:1) to obtain the target compound (yield: 96%).

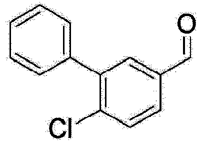

¹H NMR (400 MHz, CDCl₃) δ 10.05 (s, 1H), 7.88 (s, 1H), 7.83 (d, *J =* 8.0 Hz, 1H), 7.68 (d, *J* = 8.1 Hz, 1H), 7.55-7.43 (m, 5H).

¹³C NMR (100 MHz, CDCl₃) δ 190.96, 141.56, 139.21, 138.09, 135.02, 132.62, 130.90, 129.33, 129.05, 128.32, 128.27.

### Step 3: Preparation of 1-((6-chloro-[1,1'-biphenyl]-3-yl)methyl)pyrrolidine

After dissolving 6-chloro-[1,1'-biphenyl]-3-carbaldehyde (1.0 mmol) and pyrrolidine (2.0 mmol) in methanol in a reaction vessel, acetic acid (1.0 mmol) was added and the reaction mixture was stirred at room temperature for 2 hours. After adding sodium triacetoxyborohydride (3.0 mmol), the reaction mixture was stirred for 24 hours. After adding a saturated sodium bicarbonate solution, the reaction mixture was extracted with dichloromethane. The obtained organic layer was dried with anhydrous magnesium sulfate and then filtered. After concentrating the filtrate under reduced pressure, the obtained concentrate was separated by column chromatography (hexane:ethyl acetate = 3:1) to obtain compound 12 (yield: 44%).

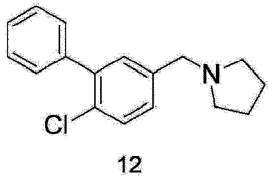

¹H NMR (400 MHz, CDCl₃) δ 7.51-7.38 (m, 6H), 7.35 (d, *J* = 2.2 Hz, 1H), 7.29 (dd, *J* = 8.0, 2.4 Hz, 1H), 3.65 (s, 2H), 2.60-2.51 (m, 4H), 1.86-1.78 (m, 4H).

¹³C NMR (100 MHz, CDCl₃) δ 140.19, 139.48, 138.37, 131.75, 130.78, 129.73, 129.52, 128.96, 127.99, 127.54, 59.93, 54.19, 23.50.

### Example 13: Preparation of 1-((2',6-dichloro-[1,1'-biphenyl]-3-yl)methyl)pyrrolidine

Compound 13 (yield: 58%) was obtained using the compound 2',6-dichloro-[1,1'-biphenyl]-3-carbaldehyde (yield: 87%) prepared in the same manner as in Example 12 using 4-chloro-3-iodobenzaldehyde (1.0 mmol), 2-chlorophenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

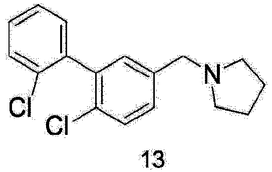

¹H NMR (400 MHz, CDCl₃) δ 7.49-7.45 (m, 1H), 7.41 (d, *J* = 8.2 Hz, 1H), 7.34-7.27 (m, 4H), 7.25 (d, *J* = 2.1 Hz, 1H), 3.62 (s, 2H), 2.55-2.49 (m, 4H), 1.79 (m, 4H).

¹³C NMR (100 MHz, CDCl₃) δ 138.43, 138.09, 138.01, 133.55, 131.73, 131.52, 131.29, 129.64, 129.41, 129.18, 129.15, 126.44, 59.85, 54.14, 23.51.

### Example 14: Preparation of 1-((3',6-dichloro-[1,1'-biphenyl]-3-yl)methyl)pyrrolidine

Compound 14 (yield: 60%) was obtained using the compound 3',6-dichloro-[1,1'-biphenyl]-3-carbaldehyde (yield: 85%) prepared in the same manner as in Example 12 using 4-chloro-3-iodobenzaldehyde (1.0 mmol), 3-chlorophenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

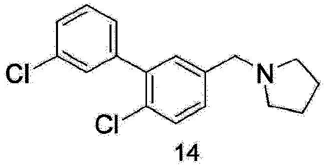

¹H NMR (400 MHz, CDCl₃) δ 7.45-7.42 (m, 1H), 7.40 (d, *J* = 7.9 Hz, 1H), 7.36-7.31 (m, 3H), 7.30-7.25 (m, 2H), 3.61 (s, 2H), 2.55-2.47 (m, 4H), 1.83-1.75 (m, 4H).

¹³C NMR (100 MHz, CDCl₃) δ 141.16, 138.82, 138.64, 133.86, 131.49, 130.64, 129.82, 129.59, 129.42, 129.22, 127.81, 127.66, 59.88, 54.21, 23.51.

### Example 15: Preparation of 1-((4',6-dichloro-[1,1'-biphenyl]-3-yl)methyl)pyrrolidine

Compound 15 (yield: 42%) was obtained using the compound 4',6-dichloro-[1,1'-biphenyl]-3-carbaldehyde (yield: 77%) prepared in the same manner as in Example 12 using 4-chloro-3-iodobenzaldehyde (1.0 mmol), 4-chlorophenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

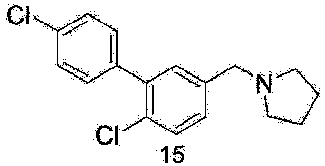

¹H NMR (400 MHz, CDCl₃) δ 7.41-7.37 (m, 5H), 7.29-7.24 (m, 2H), 3.61 (s, 2H), 2.55-2.48 (m, 4H), 1.82-1.75 (m, 4H).

¹³C NMR (100 MHz, CDCl₃) δ 139.00, 138.62, 137.84, 133.64, 131.49, 130.88, 130.67, 129.82, 129.26, 128.22, 59.89, 54.21, 23.51.

### Example 16: Preparation of 1-((6-chloro-2'-methyl-[1,1'-biphenyl]-3-yl)methyl)pyrrolidine

Compound 16 (yield: 62%) was obtained using the compound 6-chloro-2'-methyl-[1,1'-biphenyl]-3-carbaldehyde (yield: 97%) prepared in the same manner as in Example 12 using 4-chloro-3-iodobenzaldehyde (1.0 mmol), 2-methylphenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

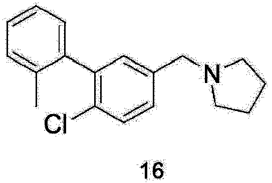

¹H NMR (400 MHz, CDCl₃) δ 7.43 (d, *J* = 8.2 Hz, 1H), 7.37-7.29 (m, 1H), 7.33-7.22 (m, 3H), 7.23 (d, *J* = 2.2 Hz, 1H), 7.18 (dd, *J* = 7.4, 1.4 Hz, 1H), 3.65 (s, 2H), 2.60-2.50 (m, 4H), 2.15 (s, 3H), 1.82 (m, 4H).

¹³C NMR (100 MHz, CDCl₃) δ 140.27, 139.44, 137.99, 136.24, 131.71, 131.40, 129.76, 129.45, 129.15, 129.04, 127.85, 125.46, 59.88, 54.13, 23.49, 19.84.

### Example 17: Preparation of 1-((6-chloro-3'methyl-[1,1'-biphenyl]-3-yl)methyl)pyrrolidine

Compound 17 (yield: 60%) was obtained using the compound 6-chloro-3'-methyl-[1,1'-biphenyl]-3-carbaldehyde (yield: 97%) prepared in the same manner as in Example 12 using 4-chloro-3-iodobenzaldehyde (1.0 mmol), 3-methylphenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

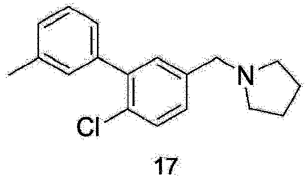

¹H NMR (400 MHz, CDCl₃) δ 7.32 (d, *J* = 8.1 Hz, 1H), 7.24-7.15 (m, 5H), 7.11 (d, *J* = 7.2 Hz, 1H), 3.54 (s, 2H), 2.44 (m, 4H), 2.33 (s, 3H), 1.76-1.68 (m, 4H).

¹³C NMR (100 MHz, CDCl₃) δ 140.32, 139.41, 138.20, 137.61, 131.75, 130.79, 130.17, 129.68, 128.88, 128.29, 127.85, 126.60, 59.93, 54.17, 23.49, 21.49.

### Example 18: Preparation of 1-((6-chloro-4'methyl-[1,1'-biphenyl]-3-ylmethyl)pyrrolidine

Compound 18 (yield: 68%) was obtained using the compound 6-chloro-4'-methyl-[1,1'-biphenyl]-3-carbaldehyde (yield: 64%) prepared in the same manner as in Example 12 using 4-chloro-3-iodobenzaldehyde (1.0 mmol), 4-methylphenylboronic acid (1.2 mmol), PdCl₂(PPh₃)₂ (0.1 mmol) and Na₂CO₃ (4.0 mmol), pyrrolidine (2.0 mmol), acetic acid (1.0 mmol) and sodium triacetoxyborohydride (3.0 mmol).

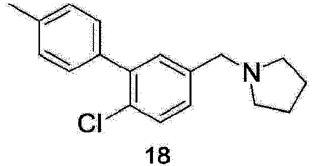

¹H NMR (400 MHz, CDCl₃) δ 7.43 (d, *J* = 8.1 Hz, 1H), 7.39 (d, *J* = 7.8 Hz, 2H), 7.33 (d, *J* = 2.1 Hz, 1H), 7.29-7.25 (m, 3H), 3.65 (s, 2H), 2.55 (m, 4H), 2.44 (s, 3H), 1.87-1.76 (m, 4H).

¹³C NMR (100 MHz, CDCl₃) δ 140.15, 138.20, 137.31, 136.57, 131.78, 130.85, 129.71, 129.38, 128.78, 128.72, 59.90, 54.15, 23.49, 21.27.

### Example 19: Preparation of 1-((6-chloro-2'-methoxy-[1,1'-biphenyl]-3-yl)methyl)pyrrolidine

Compound 19 (yield: 72%) was obtained using the compound 6-chloro-2'-methoxy-[1,1'-biphenyl]-3-carbaldehyde (yield: 55%) prepared in the same manner as in Example 12 using 4-chloro-3-iodobenzaldehyde (1.0 mmol), 2-methoxyphenylboronicacid (1.2 mmol), Pd(PPh₃)₄(0.1 mmol) and Na₂CO₃ (4.0 mmol), pyrrolidine (2.0 mmol), acetic acid (1.0 mmol) and sodium triacetoxyborohydride (3.0 mmol).

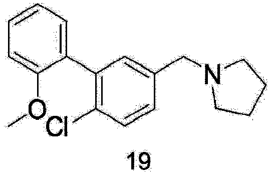

¹H NMR (400 MHz, CDCl₃) δ 7.41-7.33 (m, 2H), 7.28-7.22 (m, 2H), 7.22-7.17 (dd, *J* = 7.5, 1.8 Hz, 1H), 7.03-6.98 (td, *J* = 7.5, 1.1 Hz, 1H), 6.98-6.94 (dd, *J* = 7.2, 1.2 Hz, 1H), 3.77 (s, 3H), 3.61 (s, 2H), 2.56-2.46 (m, 4H), 1.83-1.74 (m, 4H).

¹³C NMR (100 MHz, CDCl₃) δ 156.80, 137.78, 137.40, 132.23, 132.09, 131.10, 129.30, 129.07, 128.97, 128.67, 120.32, 111.01, 59.94, 55.64, 54.16, 23.51.

### Example 20: Preparation of 1-((6-chloro-3'methoxy-[1,1'-biphenyl]-3-yl)methyl)pyrrolidine

Compound 20 (yield: 86%) was obtained using the compound 6-chloro-3'-methoxy-[1,1'-biphenyl]-3-carbaldehyde (yield: 45%) prepared in the same manner as in Example 12 using 4-chloro-3-iodobenzaldehyde (1.0 mmol), 3-methoxyphenylboronicacid (1.2 mmol), Pd(PPh₃)₄(0.1 mmol) and Na₂CO₃ (4.0 mmol), pyrrolidine (2.0 mmol), acetic acid (1.0 mmol) and sodium triacetoxyborohydride (3.0 mmol).

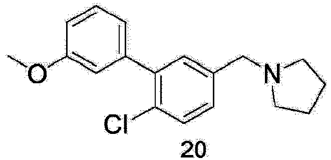

¹H NMR (400 MHz, CDCl₃) δ 7.45 (d, *J* = 8.1 Hz, 1H), 7.40-7.35 (m, 2H), 7.33-7.29 (dd, *J* = 8.2, 2.2 Hz, 1H), 7.10-7.06 (dt, *J* = 7.6, 1.2 Hz, 1H), 7.05 (t, *J* = 2.1 Hz, 1H), 6.99-6.93 (dd, *J* = 8.2, 2.2 Hz, 1H), 3.87 (s, 3H), 3.66 (s, 2H), 2.56 (m, 4H), 1.83 (m, 4H).

¹³C NMR (100 MHz, CDCl₃) δ 159.20, 140.80, 140.07, 138.19, 131.69, 130.80, 129.77, 129.05, 129.02, 121.99, 115.24, 113.15, 59.87, 55.33, 54.18, 23.51.

### Example 21: Preparation of 1-((6-chloro-4'-methoxy-[1,1'-biphenyl]-3-yl)methyl)pyrrolidine

Compound 21 (yield: 84%) was obtained using the compound 6-chloro-4'-methoxy-[1,1'-biphenyl]-3-carbaldehyde (yield: 55%) prepared in the same manner as in Example 12 using 4-chloro-3-iodobenzaldehyde (1.0 mmol), 4-methoxyphenylboronicacid (1.2 mmol), Pd(PPh₃)₄(0.1 mmol) and Na₂CO₃ (4.0 mmol), pyrrolidine (2.0 mmol), acetic acid (1.0 mmol) and sodium triacetoxyborohydride (3.0 mmol).

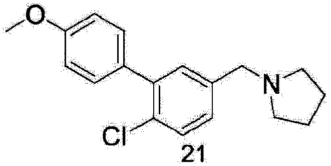

¹H NMR (400 MHz, CDCl₃) δ 7.45-7.39 (m, 3H), 7.33 (d, *J* = 2.2 Hz, 1H), 7.28-7.24 (dd, *J* = 8.1, 2.2 Hz, 1H), 7.01-6.97 (m, 2H), 3.88 (s, 3H), 3.64 (s, 2H), 2.59-2.52 (m, 4H), 1.85-1.79 (m, 4H).

¹³C NMR (100 MHz, CDCl₃) δ 159.08, 139.81, 138.18, 131.86, 131.77, 130.92, 130.68, 129.73, 128.65, 113.43, 59.92, 55.30, 54.17, 23.49.

### Example 22: Preparation of 1-((6-chloro-2,6'-dimethoxy-[1,1'-biphenyl]-3-ylmethyl)pyrrolidine

Compound 22 (yield: 70%) was obtained using the compound 6-chloro-2',6'-dimethoxy-[1,1'-biphenyl]-3-carbaldehyde (yield: 92%) prepared in the same manner as in Example 12 using 4-chloro-3-iodobenzaldehyde (1.0 mmol), 2,6-dimethoxyphenylboronic acid (1.2 mmol), Pd(PPh₃)₄ (0.1 mmol) and Na₂CO₃ (4.0 mmol), pyrrolidine (2.0 mmol), acetic acid (1.0 mmol) and sodium triacetoxyborohydride (3.0 mmol).

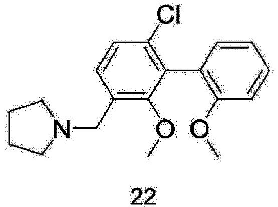

¹H NMR (400 MHz, CDCl₃) δ 7.40 (d, *J* = 8.1 Hz, 1H), 7.31 (t, *J* = 8.3 Hz, 1H), 7.24 (dd, *J* = 8.2, 2.1 Hz, 1H), 7.20 (d, *J* = 2.2 Hz, 1H), 6.64 (d, *J* = 8.4 Hz, 2H), 3.72 (s, 6H), 3.62 (s, 2H), 2.56-2.50 (m, 4H), 1.81-1.75 (m, 4H).

¹³C NMR (100 MHz, CDCl₃) δ 157.85, 137.14, 133.43, 133.02, 133.00, 129.39, 128.93, 128.89, 117.17, 104.11, 59.88, 55.99, 53.98, 23.49.

### Example 23: Preparation of 2-([1,1'-biphenyl]-3-yl)-4,5-dihydro-1H-imidazole

### Step 1: Preparation of [1,1'-biphenyl]-3-carbaldehyde

After dissolving 3-bromobenzaldehyde (1.0 mmol), phenylboronic acid (1.2 mmol), PdCl₂(PPh₃)₂ (0.1 mmol) and Na₂CO₃ (4.0 mmol) in tetrahydrofuran (10 mL) in a reaction vessel, the reaction mixture was refluxed by heating at 70 °C for 24 hours. After cooling to room temperature and adding distilled water, the reaction mixture was extracted with dichloromethane. The obtained organic layer was dried with anhydrous magnesium sulfate and then filtered. After concentrating the filtrate under reduced pressure, the obtained concentrate was separated by column chromatography (hexane:ethyl acetate = 20:1 ) to obtain the target compound (yield: 91%).

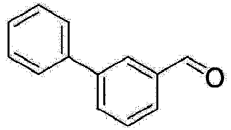

¹H NMR (400 MHz, CDCl₃) δ 10.12 (s, 1H), 8.13 (t, *J* = 1.8 Hz, 1H), 7.89 (dd, *J* = 7.7, 1.8 Hz, 2H), 7.68-7.61 (m, 3H), 7.54-7.48 (m, 2H), 7.46-7.41 (m, 1H).

¹³C NMR (100 MHz, CDCl₃) δ 192.35, 142.19, 139.72, 136.95, 133.08, 129.52, 129.03, 128.65, 128.22, 128.04, 127.17.

### Step 2: Preparation of 2-([1,1'-biphenyl]-3-yl)-4,5-dihydro-1H-imidazole

After dissolving the [1,1'-biphenyl]-3-carbaldehyde prepared in the step 1 (1.0 mmol) and ethylenediamine (1.2 mmol) in dichloromethane in a reaction vessel, the reaction mixture was stirred at 0 °C for 1 hour. After adding N-bromosuccinimide (1.2 mmol) at the same temperature, the reaction mixture was stirred for 24 hours. After adding a saturated sodium bicarbonate solution, the reaction mixture was extracted with dichloromethane. The obtained organic layer was dried with anhydrous magnesium sulfate and then filtered. After concentrating the filtrate under reduced pressure, the obtained concentrate was separated by column chromatography (dichloromethane:methanol = 20:1) to obtain compound 23 (yield: 53%).

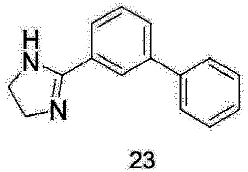

¹H NMR (400 MHz, DMSO-d₆) δ 8.12 (t, *J* = 1.8 Hz, 1H), 7.87-7.82 (dt, *J* = 7.7, 1.4 Hz, 1H), 7.78-7.74 (dt, *J* = 7.8, 1.5 Hz, 1H), 7.74-7.69 (m, 2H), 7.56-7.46 (m, 3H), 7.44-7.36 (m, 1H), 3.64 (s, 4H).

¹³C NMR (100 MHz, DMSO-d₆) δ 163.99, 140.54, 140.16, 131.73, 129.46, 129.33, 128.86, 128.17, 127.22, 126.64, 125.81, 50.12.

### Example 24: Preparation of 2-(2'-chloro-[1,1'-biphenyl]-3-yl)-4,5-dihydro-1H-imidazole

Compound 24 (yield: 90%) was obtained using the compound 2'-chloro-[1,1'-biphenyl]-3-carbaldehyde (yield: 85%) prepared in the same manner as in Example 23 using 3-bromobenzaldehyde (1.0 mmol), 2-chlorophenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

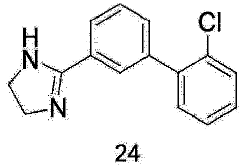

¹H NMR (400 MHz, DMSO-d₆) δ 7.91-7.86 (m, 2H), 7.61-7.57 (m, 1H), 7.57-7.52 (m, 2H), 7.47-7.42 (m, 3H), 3.64 (s, 4H).

¹³C NMR (100 MHz, DMSO-d₆) δ 163.88, 139.75, 139.14, 131.97, 131.78, 131.68, 130.62, 130.33, 129.93, 128.68, 128.46, 128.05, 126.99, 49.81.

### Example 25: Preparation of 2-(3'-chloro-[1,1'-biphenyl]-3-yl)-4,5-dihydro-1H-imidazole

Compound 25 (yield: 65%) was obtained using the compound 3'-chloro-[1,1'-biphenyl]-3-carbaldehyde (yield: 86%) prepared in the same manner as in Example 23 using 3-bromobenzaldehyde (1.0 mmol), 3-chlorophenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

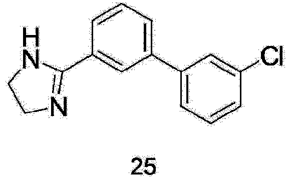

¹H NMR (400 MHz, DMSO-d₆) δ 8.17 (t, *J* = 1.8 Hz, 1H), 7.95-7.90 (m, 1H), 7.84 (dt, *J* = 8.0, 1.4 Hz, 1H), 7.81 (t, *J* = 1.9 Hz, 1H), 7.71 (dt, *J* = 7.6, 1.4 Hz, 1H), 7.54 (dt, *J* = 16.0, 7.8 Hz, 2H), 7.46 (dt, *J* = 8.2, 1.4 Hz, 1H), 3.71 (s, 4H).

¹³C NMR (100 MHz, DMSO-d₆) δ 164.03, 142.03, 139.07, 134.32, 131.30, 130.42, 129.63, 129.62, 128.11, 127.50, 126.97, 126.15, 125.94, 49.24.

### Example 26: Preparation of 2-(4'-chloro-[1,1'-biphenyl]-3-yl)-4,5-dihydro-1H-imidazole

Compound 26 (yield: 58%) was obtained using the compound 4'-chloro-[1,1'-biphenyl]-3-carbaldehyde (yield: 78%) prepared in the same manner as in Example 23 using 3-bromobenzaldehyde (1.0 mmol), 4-chlorophenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

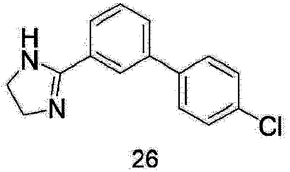

¹H NMR (400 MHz, DMSO-d₆) δ 8.11 (t, *J* = 1.8 Hz, 1H), 7.88-7.84 (dt, *J* = 7.7, 1.4 Hz, 1H), 7.79-7.73 (m, 3H), 7.58-7.51 (m, 3H), 3.65 (s, 4H).

¹³C NMR (100 MHz, DMSO-d₆) δ 163.89, 139.19, 138.91, 133.10, 131.62, 129.47, 129.43, 128.98, 128.88, 127.00, 125.74, 50.01.

### Example 27: Preparation of 2-(2'-methyl-[1,1'-biphenyl]-3-yl)-4,5-dihydro-1H-imidazole

Compound 27 (yield: 27%) was obtained using the compound 2'-methyl-[1,1'-biphenyl]-3-carbaldehyde (yield: 93%) prepared in the same manner as in Example 23 using 3-bromobenzaldehyde (1.0 mmol), 2-methylphenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

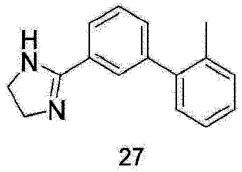

¹H NMR (400 MHz, DMSO-d₆) δ 7.89-7.84 (dt, *J* = 7.7, 1.6 Hz, 1H), 7.82-7.79 (t, *J* = 1.7 Hz, 1H), 7.54-7.47 (t, *J* = 7.6 Hz, 1H), 7.47-7.42 (dt, *J* = 7.6, 1.5 Hz, 1H), 7.33-7.21 (m, 4H), 3.64 (s, 4H), 2.23 (s, 3H).

¹³C NMR (100 MHz, DMSO-d₆) δ 164.04, 141.69, 141.20, 135.19, 131.42, 130.83, 130.54, 129.97, 128.66, 128.14, 128.03, 126.46, 126.26, 49.77, 20.58.

### Example 28: Preparation of 2-(3'-methyl-[1,1'-biphenyl]-3-yl)-4,5-dihydro-1H-imidazole

Compound 28 (yield: 54%) was obtained using the compound 3'-methyl-[1,1'-biphenyl]-3-carbaldehyde (yield: 95%) prepared in the same manner as in Example 23 using 3-bromobenzaldehyde (1.0 mmol), 3-methylphenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

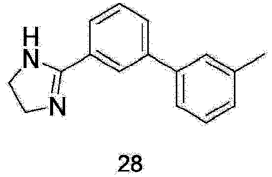

¹H NMR (400 MHz, DMSO-d₆) δ 8.10 (t, *J* = 1.8 Hz, 1H), 7.85-7.81 (dt, *J* = 7.8, 1.4 Hz, 1H), 7.77-7.72 (dt, *J* = 7.9, 1.4 Hz, 1H), 7.55-7.47 (m, 3H), 7.38 (t, *J* = 7.6 Hz, 1H), 7.21 (d, *J* = 7.5 Hz, 1H), 3.64 (s, 4H), 2.40 (s, 3H).

¹³C NMR (100 MHz, DMSO-d₆) δ 163.99, 140.62, 140.11, 138.62, 131.68, 129.35, 129.27, 128.83, 128.80, 127.85, 126.54, 125.77, 124.33, 50.21, 21.58.

### Example 29: Preparation of 2-(4'-methyl-[1,1'-biphenyl]-3-yl)-4,5-dihydro-1H-imidazole

Compound 29 (yield: 47%) was obtained using the compound 4'-methyl-[1,1'-biphenyl]-3-carbaldehyde (yield: 96%) prepared in the same manner as in Example 23 using 3-bromobenzaldehyde (1.0 mmol), 4-methylphenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

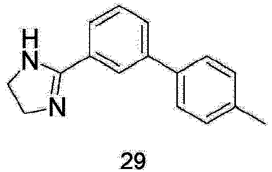

¹H NMR (400 MHz, DMSO-d₆) δ 8.12-8.09 (t, *J* = 1.8 Hz, 1H), 7.84-7.79 (dt, *J* = 7.8, 1.4 Hz, 1H), 7.78-7.73 (dt, *J* = 8.0, 1.4 Hz, 1H), 7.65-7.59 (m, 2H), 7.51 (t, *J* = 7.7 Hz, 1H), 7.33-7.28 (m, 2H), 3.66 (s, 4H), 2.36 (s, 3H).

¹³C NMR (100 MHz, DMSO-d₆) δ 164.11, 140.47, 137.57, 137.14, 131.12, 130.06, 129.35, 128.85, 127.03, 126.40, 125.60, 49.75, 21.15.

### Example 30: Preparation of 2-(2'-methoxy-[1,1'-biphenyl]-3-yl)-4,5-dihydro-1H-imidazole

Compound 30 (yield: 82%) was obtained using the compound 2'-methoxy-[1,1'-biphenyl]-3-carbaldehyde (yield: 92%) prepared in the same manner as in Example 23 using 3-bromobenzaldehyde (1.0 mmol), 2-methoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

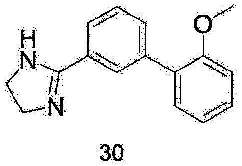

¹H NMR (400 MHz, DMSO-d₆) δ 7.91 (t, *J* = 1.8 Hz, 1H), 7.80-7.76 (dt, *J* = 7.7, 1.4 Hz, 1H), 7.59-7.54 (dt, *J* = 7.7, 1.4 Hz, 1H), 7.48-7.42 (t, *J* = 7.7 Hz, 1H), 7.41-7.35 (ddd, *J* = 8.2, 7.3, 1.8 Hz, 1H), 7.34 -7.30 (dd, *J* = 7.5, 1.8 Hz, 1H), 7.16-7.11 (dd, *J* = 8.3, 1.1 Hz, 1H), 7.08-7.03 (td, *J* = 7.4, 1.1 Hz, 1H), 3.77 (s, 3H), 3.62 (s, 4H).

¹³C NMR (100 MHz, DMSO-d₆) δ 164.14, 156.58, 138.57, 131.56, 130.93, 130.90, 129.80, 129.60, 128.43, 128.29, 126.05, 121.25, 112.21, 55.99, 50.05.

### Example 31: Preparation of 2-(3'-methoxy-[1,1'-biphenyl]-3-yl)-4,5-dihydro-1H-imidazole

Compound 31 (yield: 73%) was obtained using the compound 3'-methoxy-[1,1'-biphenyl]-3-carbaldehyde (yield: 96%) prepared in the same manner as in Example 23 using 3-bromobenzaldehyde (1.0 mmol), 3-methoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

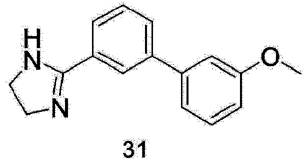

¹H NMR (400 MHz, DMSO-d₆) δ 8.08 (t, *J* = 1.7 Hz, 1H), 7.88-7.82 (dt, *J* = 7.7, 1.3 Hz, 1H), 7.79-7.74 (ddd, *J* = 7.8, 1.9, 1.1 Hz, 1H), 7.51 (t, *J* = 7.7 Hz, 1H), 7.41 (t, *J* = 7.9 Hz, 1H), 7.31-7.25 (ddd, *J* = 7.7, 1.7, 0.9 Hz, 1H), 7.25-7.23 (m, 1H), 7.00-6.95 (ddd, *J* = 8.2, 2.6, 0.9 Hz, 1H), 3.84 (s, 3H), 3.64 (s, 4H).

¹³C NMR (100 MHz, DMSO-d₆) δ 163.94, 160.27, 141.66, 140.42, 131.67, 130.53, 129.28, 128.98, 126.79, 125.83, 119.55, 113.73, 112.75, 55.64, 50.07.

### Example 32: Preparation of 2-(4'-methoxy-[1,1'-biphenyl]-3-yl)-4,5-dihydro-1H-imidazole

Compound 32 (yield: 60%) was obtained using the compound 4'-methoxy-[1,1'-biphenyl]-3-carbaldehyde (yield: 91%) prepared in the same manner as in Example 23 using 3-bromobenzaldehyde (1.0 mmol), 4-methoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

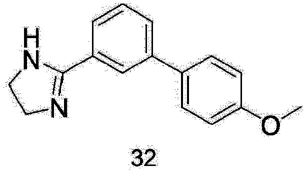

¹H NMR (400 MHz, DMSO-d₆) δ 8.07 (t, *J* = 1.8 Hz, 1H), 7.82-7.77 (dt, *J* = 7.7, 1.4 Hz, 1H), 7.74-7.69 (dt, *J* = 7.9, 1.4 Hz, 1H), 7.69-7.63 (m, 2H), 7.48 (t, *J* = 7.7 Hz, 1H), 7.08-7.02 (m, 2H), 3.81 (s, 3H), 3.64 (s, 4H).

¹³C NMR (100 MHz, DMSO-d₆) δ 164.08, 159.56, 140.17, 132.46, 131.64, 129.24, 128.33, 128.30, 125.93, 125.27, 114.88, 55.65, 50.06.

### Example 33: Preparation of 2-(2',6'-dimethoxy-[1,1'-biphenyl]-3-yl)-4,5-dihydro-1H-imidazole

Compound 33 (yield: 55%) was obtained using the compound 2',6'-dimethoxy-[1,1'-biphenyl]-3-carbaldehyde (yield: 89%) prepared in the same manner as in Example 23 using 3-bromobenzaldehyde (1.0 mmol), 2,6-dimethoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

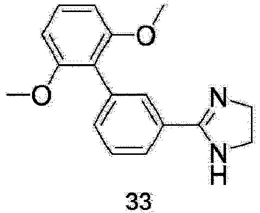

¹H NMR (400 MHz, DMSO-d₆) δ 7.78-7.74 (dt, *J* = 7.8, 1.5 Hz, 1H), 7.68 (t, *J* = 1.7 Hz, 1H), 7.42 (t, *J* = 7.7 Hz, 1H), 7.36-7.28 (m, 2H), 6.76 (d, *J* = 8.4 Hz, 2H), 3.66 (s, 6H), 3.62 (s, 4H).

¹³C NMR (100 MHz, DMSO-d₆) δ 164.31, 157.61, 134.76, 133.36, 130.02, 129.98, 129.70, 128.00, 125.87, 118.56, 104.81, 56.16, 49.71.

### Example 34: Preparation of 2-(6-chloro-[1,1'-biphenyl]-3-yl)-4,5-dihydro-1H-imidazole

### Step 1: Preparation of 4-chloro-3-iodobenzaldehyde

The target compound was synthesized in the same manner as in the step 1 of Example 12.

### Step 2: Preparation of 6-chloro-[1,1'-biphenyl]-3-carbaldehyde

The target compound was synthesized in the same manner as in the step 2 of Example 12.

### Step 3: Preparation of 2-(6-chloro-[1,1'-biphenyl]-3-yl)-4,5-dihydro-1H-imidazole

After dissolving 6-chloro-[1,1'-biphenyl]-3-carbaldehyde (1.0 mmol) and ethylenediamine (1.2 mmol) in dichloromethane in a reaction vessel, the reaction mixture was stirred at 0 °C for 1 hour. After adding N-bromosuccinimide (1.2 mmol) at the same temperature, the reaction mixture was stirred for 24 hours. After adding a saturated sodium bicarbonate solution, the reaction mixture was extracted with dichloromethane. The obtained organic layer was dried with anhydrous magnesium sulfate and then filtered. After concentrating the filtrate under reduced pressure, the obtained concentrate was separated by column chromatography (dichloromethane:methanol = 20:1) to obtain compound 34 (yield: 34%).

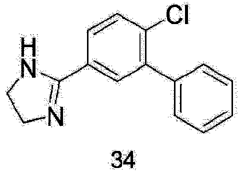

¹H NMR (400 MHz, DMSO-d₆) δ 7.88-7.84 (m, 2H), 7.67-7.63 (m, 1H), 7.53-7.43 (m, 5H), 3.64 (s, 4H).

¹³C NMR (100 MHz, DMSO-d₆) δ 163.01, 140.09, 138.73, 133.68, 130.49, 130.33, 129.87, 129.69, 128.77, 128.47, 128.17, 49.92.

### Example 35: Preparation of 2-(2',6-dichloro-[1,1'-biphenyl]-3-yl)-4,5-dihydro-1H-imidazole

Compound 35 (yield: 50%) was obtained using the compound 2',6-dichloro-[1,1'-biphenyl]-3-carbaldehyde (yield: 87%) prepared in the same manner as in Example 34 using 4-chloro-3-iodobenzaldehyde (1.0 mmol), 2-chlorophenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

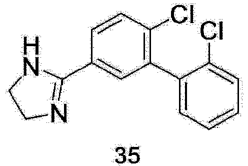

¹H NMR (400 MHz, DMSO-d₆) δ 7.92 (dd, *J* = 8.4, 2.2 Hz, 1H), 7.79 (d, *J* = 2.1 Hz, 1H), 7.67 (d, *J* = 8.4 Hz, 1H), 7.64-7.58 (m, 1H), 7.51-7.44 (m, 2H), 7.41-7.36 (m, 1H), 3.63 (s, 4H).

¹³C NMR (100 MHz, DMSO-d₆) δ 162.84, 138.08, 137.74, 134.85, 132.82, 131.73, 130.60, 130.26, 129.78, 129.76, 129.61, 128.92, 127.78, 49.93.

### Example 36: Preparation of 2-(3',6-dichloro-[1,1'-biphenyl]-3-yl)-4,5-dihydro-1H-imidazole

Compound 36 (yield: 49%) was obtained using the compound 3',6-dichloro-[1,1'-biphenyl]-3-carbaldehyde (yield: 85%) prepared in the same manner as in Example 34 using 4-chloro-3-iodobenzaldehyde (1.0 mmol), 3-chlorophenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

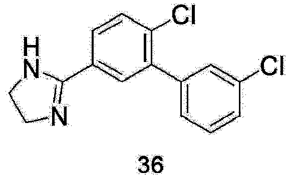

¹H NMR (400 MHz, DMSO-d₆) δ 7.93-7.89 (m, 2H), 7.73-7.68 (m, 1H), 7.58-7.51 (m, 3H), 7.49-7.44 (m, 1H), 3.70 (s, 4H).

¹³C NMR (100 MHz, DMSO-d₆) δ 163.10, 140.49, 138.74, 134.29, 133.46, 130.69, 130.68, 130.61, 129.45, 128.87, 128.68, 128.58, 128.57, 49.13.

### Example 37: Preparation of 2-(4',6-dichloro-[1,1'-biphenyl]-3-yl)-4,5-dihydro-1H-imidazole

Compound 37 (yield: 43%) was obtained using the compound 4',6-dichloro-[1,1'-biphenyl]-3-carbaldehyde (yield: 77%) prepared in the same manner as in Example 34 using 4-chloro-3-iodobenzaldehyde (1.0 mmol), 4-chlorophenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

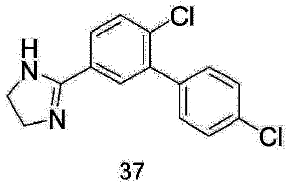

¹H NMR (400 MHz, DMSO-d₆) δ 7.89-7.86 (m, 2H), 7.67-7.64 (m, 1H), 7.58-7.54 (m, 2H), 7.52-7.48 (m, 2H), 3.64 (s, 4H).

¹³C NMR (100 MHz, DMSO-d₆) δ 162.96, 138.84, 137.46, 133.71, 133.44, 131.59, 130.42, 129.82, 128.81, 128.52, 49.83.

### Example 38: Preparation of 2-(6-chloro-2'-methyl-[1,1'-biphenyl]-3-yl)-4,5-dihydro-1H-imidazole

Compound 38 (yield: 57%) was obtained using the compound 6-chloro-2'-methyl-[1,1'-biphenyl]-3-carbaldehyde (yield: 97%) prepared in the same manner as in Example 34 using 4-chloro-3-iodobenzaldehyde (1.0 mmol), 2-methylphenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

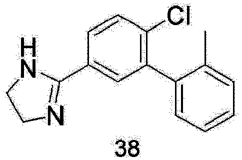

¹H NMR (400 MHz, DMSO-d₆) δ 7.89 (dd, J = 8.4, 2.2 Hz, 1H), 7.74 (d, J = 2.2 Hz, 1H), 7.66 (d, *J* = 8.4 Hz, 1H), 7.36-7.32 (m, 2H), 7.32-7.26 (m, 1H), 7.17-7.12 (m, 1H), 3.65 (s, 4H), 2.06 (s, 3H).

¹³C NMR (100 MHz, DMSO-d₆) δ 163.07, 140.29, 138.76, 135.93, 134.95, 130.34, 130.21, 129.82, 129.64, 129.10, 128.71, 128.39, 126.28, 49.59, 19.84.

### Example 39: Preparation of 2-(6-chloro-3'-methyl-[1,1'-biphenyl]-3-yl)-4,5-dihydro-1H-imidazole

Compound 39 (yield: 47%) was obtained using the compound 6-chloro-3'-methyl-[1,1'-biphenyl]-3-carbaldehyde (yield: 76%) prepared in the same manner as in Example 34 using 4-chloro-3-iodobenzaldehyde (1.0 mmol), 3-methylphenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

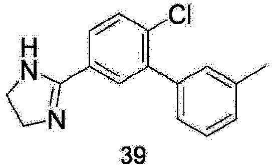

¹H NMR (400 MHz, DMSO-d₆) δ 7.87-7.81 (m, 2H), 7.66-7.61 (m, 1H), 7.38 (td, *J* = 7.3, 1.1 Hz, 1H), 7.29-7.22 (m, 3H), 3.65 (s, 4H), 2.38 (s, 3H).

¹³C NMR (100 MHz, DMSO-d₆) δ 163.08, 140.24, 138.66, 138.02, 133.82, 130.51, 130.33, 130.21, 129.56, 129.08, 128.62, 128.11, 126.80, 49.75, 21.46.

### Example 40: Preparation of 2-(6-chloro-4'-methyl-[1,1'-biphenyl]-3-yl)-4,5-dihydro-1H-imidazole

Compound 40 (yield: 76%) was obtained using the compound 6-chloro-4'-methyl-[1,1'-biphenyl]-3-carbaldehyde (yield: 64%) prepared in the same manner as in Example 34 using 4-chloro-3-iodobenzaldehyde (1.0 mmol), 4-methylphenylboronic acid (1.2 mmol), PdCl₂(PPh₃)₂ (0.1 mmol) and Na₂CO₃ (4.0 mmol), ethylenediamine (1.2 mmol) and N-bromosuccinimide (1.2 mmol).

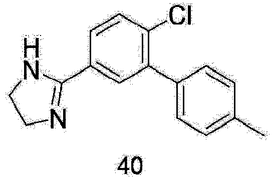

¹H NMR (400 MHz, DMSO-d₆) δ 7.89-7.82(m, 2H), 7.67-7.61 (m, 1H), 7.36 (d, *J* = 7.8 Hz, 2H), 7.29 (d, *J* = 7.9 Hz, 2H), 3.66 (s, 4H), 2.37 (s, 3H).

¹³C NMR (100 MHz, DMSO-d₆) δ 163.17, 140.11, 137.87, 135.75, 134.03, 130.57, 130.37, 129.57, 129.32, 128.04, 49.58, 21.26.

### Example 41: Preparation of 2-(6-chloro-2'-methoxy-[1,1'-biphenyl]-3-yl)-4,5-dihydro-1H-imidazole

Compound 41 (yield: 96%) was obtained using the compound 6-chloro-2'-methoxy-[1,1'-biphenyl]-3-carbaldehyde (yield: 55%) prepared in the same manner as in Example 34 using 4-chloro-3-iodobenzaldehyde (1.0 mmol), 2-methoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

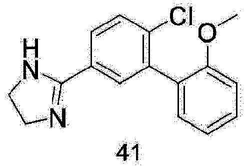

¹H NMR (400 MHz, DMSO-d₆) δ 7.85-7.81 (dd, J = 8.3, 2.2 Hz, 1H), 7.75 (d, *J* = 2.1 Hz, 1H), 7.58 (d, *J* = 8.3 Hz, 1H), 7.43 (ddd, J = 8.2, 7.4, 1.8 Hz, 1H), 7.20-7.16 (dd, J= 7.4, 1.8 Hz, 1H), 7.15-7.11 (dd, J= 8.4, 1.0 Hz, 1H), 7.07-7.02 (td, J= 7.4, 1.0 Hz, 1H), 3.73 (s, 3H), 3.62 (s, 4H).

¹³C NMR (100 MHz, DMSO-d₆) δ 163.09, 156.78, 137.79, 135.38, 130.96, 130.81, 130.30, 129.51, 129.45, 128.02, 127.78, 120.80, 111.83, 55.88, 49.96.

### Example 42: Preparation of 2-(6-chloro-3'-methoxy-[1,1'-biphenyl]-3-yl)-4,5-dihydro-1H-imidazole

Compound 42 (yield: 37%) was obtained using the compound 6-chloro-3'-methoxy-[1,1'-biphenyl]-3-carbaldehyde (yield: 45%) prepared in the same manner as in Example 34 using 4-chloro-3-iodobenzaldehyde (1.0 mmol), 3-methoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

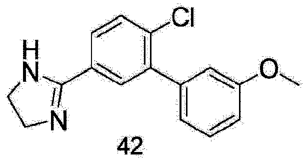

¹H NMR (400 MHz, DMSO-d₆) δ 7.88-7.83 (m, 2H), 7.65-7.59 (m, 1H), 7.44-7.37 (m, 1H), 7.05-6.99 (m, 3H), 3.81 (s, 3H), 3.62 (s, 4H).

¹³C NMR (100 MHz, DMSO-d₆) δ 162.95, 159.50, 140.09, 139.92, 133.51, 130.35, 130.28, 130.13, 129.85, 128.17, 121.99, 115.35, 113.96, 55.65, 50.11.

### Example 43: Preparation of 2-(6-chloro-4'-methoxy-[1,1'-biphenyl]-3-yl)-4,5-dihydro-1H-imidazole

Compound 43 (yield: 72%) was obtained using the compound 6-chloro-4'-methoxy-[1,1'-biphenyl]-3-carbaldehyde (yield: 55%) prepared in the same manner as in Example 34 using 4-chloro-3-iodobenzaldehyde (1.0 mmol), 4-methoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

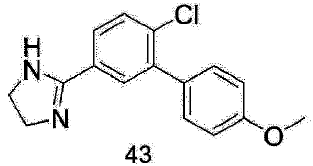

¹H NMR (400 MHz, DMSO-d₆) δ 7.87-7.79 (m, 2H), 7.63 (d, *J* = 8.3 Hz, 1H), 7.45-7.38 (m, 2H), 7.08-7.02 (m, 2H), 3.82 (s, 3H), 3.65 (s, 4H).

¹³C NMR (100 MHz, DMSO-d₆) δ 163.16, 159.51, 139.81, 133.99, 130.98, 130.85, 130.54, 130.37, 129.44, 127.80, 114.20, 55.68, 49.66.

### Example 44: Preparation of 2-(6-chloro-2',6'-dimethoxy-[1,1'-biphenyl]-3-yl)-4,5-dihydro-1H-imidazole

Compound 44 (yield: 67%) was obtained using the compound 6-chloro-2',6'-dimethoxy-[1,1'-biphenyl]-3-carbaldehyde (yield: 92%) prepared in the same manner as in Example 34 using 4-chloro-3-iodobenzaldehyde (1.0 mmol), 2,6-dimethoxyphenylboronic acid (1.2 mmol), Pd(PPh₃)₄ (0.1 mmol) and Na₂CO₃ (4.0 mmol), ethylenediamine (1.2 mmol) and N-bromosuccinimide (1.2 mmol).

¹H NMR (400 MHz, DMSO-d₆) δ 7.99-7.94 (dd, J= 8.4, 2.4 Hz, 1H), 7.87-7.81 (m, 2H), 7.43 (t, *J* = 8.4 Hz, 1H), 6.81 (d, *J* = 8.4 Hz, 2H), 3.98 (s, 4H), 3.69 (s, 6H).

¹³C NMR (100 MHz, DMSO-d₆) δ 164.29, 157.57, 140.66, 135.57, 132.84, 131.09, 130.55, 129.11, 121.70, 114.77, 104.69, 56.30, 45.21.

### [Preparation Examples]

The biphenylpyrrolidine/dihydroimidazole derivatives synthesized in the examples were prepared into various formulations.

### Preparation Example 1: Pressed tablet

5.0 mg of the active ingredient synthesized in each of the examples was sieved, mixed with 14.1 mg of lactose, 0.8 mg of crospovidone USNF and 0.1 mg of magnesium stearate, and then prepared into a tablet by pressing.

### Preparation Example 2: Wet granulation tablet

5.0 mg of the active ingredient synthesized in each of the examples was sieved, and then mixed with 16.0 mg of lactose and 4.0 mg of starch. Fine granules were prepared by dissolving 0.3 mg of polysorbate 80 in pure water and adding an adequate amount of the solution. After drying, the fine granules were sieved and mixed with 2.7 mg of colloidal silicon dioxide and 2.0 mg of magnesium stearate. A tablet was prepared by compressing the fine granules.

### Preparation Example 3: Powder and capsule

5.0 mg of the active ingredient synthesized in each of the examples was sieved, mixed with 14.8 mg of lactose, 10.0 mg of polyvinylpyrrolidone and 0.2 mg of magnesium stearate and then filled in a hard No. 5 gelatin capsule.

### Preparation Example 4: Injection

An injection was prepared using 100 mg of the compound synthesized in each of the examples as an active ingredient, 180 mg of mannitol, 26 mg of Na₂HPO₄·12H₂O and 2974 mg of distilled water.

### [Test Examples]

### Test Example 1: Measurement of inhibition of activity of 5-HT₇ serotonin receptor

### a) cAMP assay

For luminescence-based cAMP assay, HEK293 cells were cultured on a 150-mm dish. Prior to transfection, the culture medium was replaced from DMEM containing 10% FBS, 100 U/mL penicillin and 100 µg/mL streptomycin to DMEM containing 10% dialyzed FBS, 100 U/mL penicillin and 100 µg/mL streptomycin. 4 hours later, transfection was conducted using 10 µg of a human 5-HT₇R plasmid and 10 µg of a GloSensor-22F plasmid (Promega). The transfected cells were prepared on a white, flat, clear-bottom, 384-well plate (Greiner) (15,000 cells/well, 20 µL/well). 6 hours later, after removing the culture medium, the cells were treated with 20 µL of a 3% Glosensor cAMP reagent containing luciferin in a 1x HBSS, 1 M HEPES, pH 7.4 buffer. In addition, the compound of each of the examples was prepared at different concentrations in an assay buffer containing 0.1% bovine serum albumin. 30 minutes later, 10 µL of the compound solution was treated to the cells. After measuring luminescence using a Tecan microplate reader (Spark), IC₅₀ value was obtained using the Prism 8.0 program (GraphPad Software).

Table 1 shows the inhibition of the activity of the 5-HT₇ serotonin receptor (%) by the biphenylpyrrolidine derivatives of Examples 1-22, and Table 2 shows the inhibition of the activity of the 5-HT₇ serotonin receptor (%) by the biphenyldihydroimidazole derivatives of Examples 23-44.

In addition, Table 3 shows the IC₅₀ values of the biphenylpyrrolidine derivatives of the examples, and Table 4 shows the IC₅₀ values of the biphenyldihydroimidazole derivatives of the examples.

**[Table 1]**

| Compounds | % Inhibition (10 µM) |
|---|---|
| Compound 1 | 42.1 |
| Compound 2 | 62.6 |
| Compound 3 | 85.9 |
| Compound 4 | 38.1 |
| Compound 5 | 49.5 |
| Compound 6 | 38.9 |
| Compound 7 | 32.5 |
| Compound 8 | 57.1 |
| Compound 9 | 67.2 |
| Compound 10 | 37.9 |
| Compound 11 | 32.8 |
| Compound 12 | 80.8 |
| Compound 13 | 90.7 |
| Compound 14 | 51.1 |
| Compound 15 | 63.8 |
| Compound 16 | 73.3 |
| Compound 17 | 51.8 |
| Compound 18 | 73.4 |
| Compound 19 | 51.8 |
| Compound 20 | 70.1 |
| Compound 21 | 44.7 |
| Compound 22 | 42.7 |

**[Table 2]**

| Compounds | % Inhibition (10 µM) |
|---|---|
| Compound 23 | 83.3 |
| Compound 24 | 47.6 |
| Compound 25 | 50.9 |
| Compound 26 | 43.0 |
| Compound 27 | 55.3 |
| Compound 28 | 44.6 |
| Compound 29 | 35.2 |
| Compound 30 | 94.1 |
| Compound 31 | 72.5 |
| Compound 32 | 40.8 |
| Compound 33 | 32.8 |
| Compound 34 | 48.4 |
| Compound 35 | 53.8 |
| Compound 36 | 52.4 |
| Compound 37 | 43.3 |
| Compound 38 | 51.4 |
| Compound 39 | 48.1 |
| Compound 40 | 48.9 |
| Compound 41 | 44.3 |
| Compound 42 | 49.8 |
| Compound 43 | 36.8 |
| Compound 44 | 48.4 |

**[Table 3]**

| Compounds | IC₅₀ (µM) |
|---|---|
| Compound 2 | 9.51 |
| Compound 3 | 4.88 |
| Compound 9 | 6.44 |
| Compound 12 | 4.89 |
| Compound 13 | 3.20 |
| Compound 15 | 7.15 |
| Compound 16 | 6.42 |
| Compound 18 | 8.20 |
| Compound 20 | 7.48 |

**[Table 4]**

| Compounds | IC₅₀ (µM) |
|---|---|
| Compound 23 | 5.10 |
| Compound 30 | 2.58 |
| Compound 31 | 5.77 |

### b) Tango assay

Tango assay was conducted using HTLA cells in order to investigate the activity of the β-arrestin signaling system. Prior to transfection, the culture medium was replaced from DMEM containing 10% FBS, 100 U/mL penicillin, 100 µg/mL streptomycin, 2 µg/mL puromycin and 100 µg/mL hygromycin B to DMEM containing 10% dialyzed FBS, 100 U/mL penicillin and 100 µg/mL streptomycin. 4 hours later, transfection was conducted using 20 µg of a 5-HT₇R-TCS-tTA construct (5-HT₇R Tango DNA). The transfected cells were prepared on a white, flat, clear-bottom, 384-well plate (Greiner) (15,000 cells/well, 20 µL/well) using DMEM containing 10% dialyzed FBS, 100 U/mL penicillin and 100 µg/mL streptomycin. 6 hours later, after removing the culture medium, the cells were treated with 10 µL of the compound solution. After culturing for 22 hours and removing the culture medium, the cells were treated with 20 µL of a BrightGlo reagent (Promega) diluted in a 1x HBSS, 1 M HEPES, pH 7.4 buffer. After measuring luminescence using a Tecan microplate reader (Spark), IC₅₀ value was obtained using the Prism 8.0 program (GraphPad Software).

Table 5 shows the inhibition of the activity of the 5-HT₇ serotonin receptor (%) by the biphenylpyrrolidine derivatives of Examples 1-22, and Table 6 shows the inhibition of the activity of the 5-HT₇ serotonin receptor (%) by the biphenyldihydroimidazole derivatives of Examples 23-44.

In addition, Table 7 shows the IC₅₀ values of the biphenylpyrrolidine derivative of Example 1 and the biphenyldihydroimidazole derivatives of the examples.

**[Table 5]**

| Compounds | % Inhibition (10 µM) |
|---|---|
| Compound 1 | 17.5 |
| Compound 2 | 29.9 |
| Compound 3 | 33.2 |
| Compound 4 | 14.1 |
| Compound 5 | 25.0 |
| Compound 6 | 22.0 |
| Compound 7 | 20.6 |
| Compound 8 | 18.0 |
| Compound 9 | 16.7 |
| Compound 10 | 23.1 |
| Compound 11 | 5.1 |
| Compound 12 | 26.1 |
| Compound 13 | 50.5 |
| Compound 14 | 27.9 |
| Compound 15 | 34.3 |
| Compound 16 | 30.9 |
| Compound 17 | 11.4 |
| Compound 18 | 21.5 |
| Compound 19 | 19.7 |
| Compound 20 | 26.0 |
| Compound 21 | 18.6 |
| Compound 22 | 3.3 |

**[Table 6]**

| Compounds | % Inhibition (10 µM) |
|---|---|
| Compound 23 | 44.2 |
| Compound 24 | 36.0 |
| Compound 25 | 43.6 |
| Compound 26 | 23.1 |
| Compound 27 | 23.0 |
| Compound 28 | 28.8 |
| Compound 29 | 17.2 |
| Compound 30 | 40.6 |
| Compound 31 | 27.8 |
| Compound 32 | 11.9 |
| Compound 33 | 14.0 |
| Compound 34 | 31.6 |
| Compound 35 | 42.0 |
| Compound 36 | 44.8 |
| Compound 37 | 39.6 |
| Compound 38 | 31.7 |
| Compound 39 | 27.0 |
| Compound 40 | 31.3 |
| Compound 41 | 27.7 |
| Compound 42 | 23.4 |
| Compound 43 | 21.5 |
| Compound 44 | 9.8 |

**[Table 7]**

| Compounds | IC₅₀ (µM) |
|---|---|
| Compound 1 | 22.9 |
| Compound 23 | 47.9 |
| Compound 25 | 10.5 |
| Compound 30 | 39.8 |
| Compound 35 | 20.4 |
| Compound 36 | 10.0 |

### Test Example 2: Schild plot for Tango assay

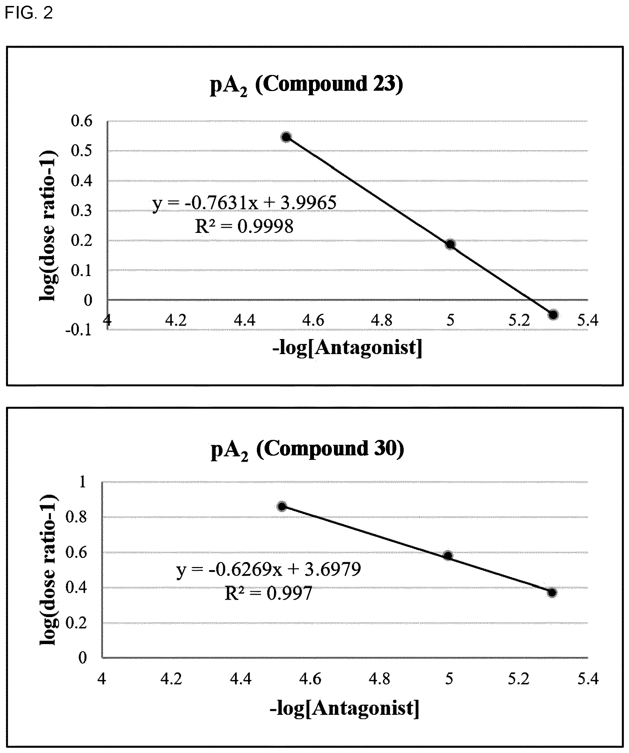

Schild plots were constructed for the Tango assay in order to investigate the effect of the compounds on the activity of the β-arrestin signaling pathway. After culturing cells and replacing the culture medium as described in b) of Test Example 1, the cells were transfected and prepared on a plate. 6 hours later, the cells were treated with 10 µL of the solution of serotonin or the compounds at final concentrations of 5 µM, 10 µM and 30 µM. After culturing for 22 hours, luminescence was measured using a Tecan microplate reader (Spark) and the Schild plot and the EC₅₀ value were obtained using the Prism 8.0 program (GraphPad Software). In addition, the pA₂ value was obtained using the Excel software. The Schild plots and pA₂ values of compound 23 and compound 30 according to the present disclosure are summarized in FIGS. 1A-1B, FIG. 2 and Table 8.

**[Table 8]**

| Compounds | pA2 |
|---|---|
| Example 23 | 5.24 |
| Example 30 | 5.90 |

It can be seen that the biphenylpyrrolidine/dihydroimidazole derivatives according to the present disclosure show antagonistic activity for the 5-HT₇ receptor. The Schild plots show that they are competitive inhibitors.

Despite the difference in the structures and physical properties of the substituents, the principles and conditions of the reactions of the examples described above apply to the compounds according to the present disclosure having substituents not described in the examples. Accordingly, it is obvious that those skilled in the art can prepare and investigate the compounds having substituents not described in the examples without special difficulty based on the foregoing disclosure and the common sense in the art.

## Claims

1. A compound for inhibiting the activity of the 5-HT₇ serotonin receptor, which is represented by Structural Formula 1 or 2, or a pharmaceutically acceptable salt thereof: 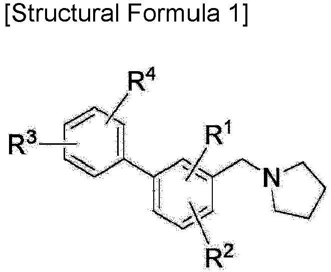 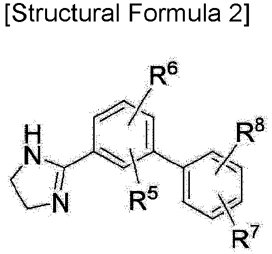 wherein
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group.

2. The compound for inhibiting the activity of the 5-HT₇ serotonin receptor or a pharmaceutically acceptable salt thereof according to claim 1, wherein the compound represented by Structural Formula 1 or 2 is represented by Structural Formula 3 or 4: 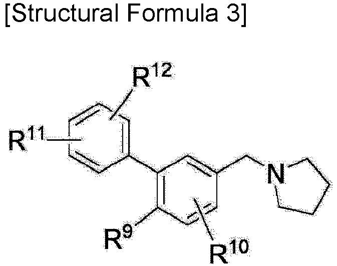 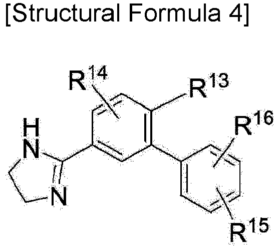 wherein
each of R⁹ to R¹⁶, which are identical to or different from each other, is independently is a hydrogen atom, a halogen group, a substituted or unsubstituted C₁-C₁₀ alkyl group or a substituted or unsubstituted C₁-C₁₀ alkoxy group.

3. The compound for inhibiting the activity of the 5-HT₇ serotonin receptor or a pharmaceutically acceptable salt thereof according to claim 2, wherein each of R⁹ to R¹⁶, which are identical to or different from each other, is independently a hydrogen atom, a chloro group, a C₁-C₄ alkyl group or a C₁-C₄ alkoxy group.

4. The compound for inhibiting the activity of the 5-HT₇ serotonin receptor or a pharmaceutically acceptable salt thereof according to claim 1, wherein the compound represented by Structural Formula 1 or 2 is any one selected from the following compounds 1-44:

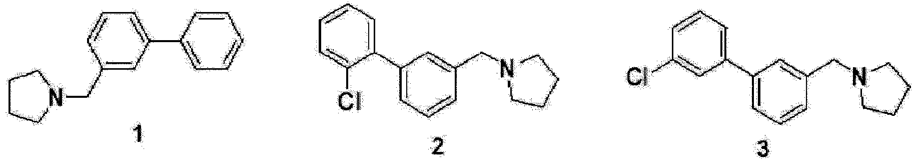

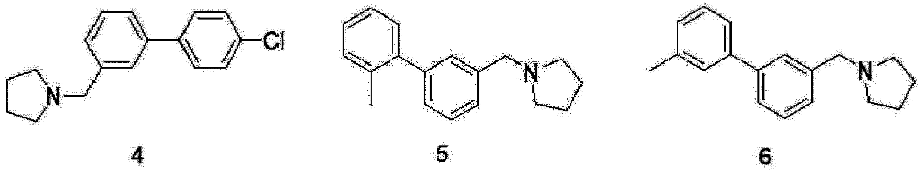

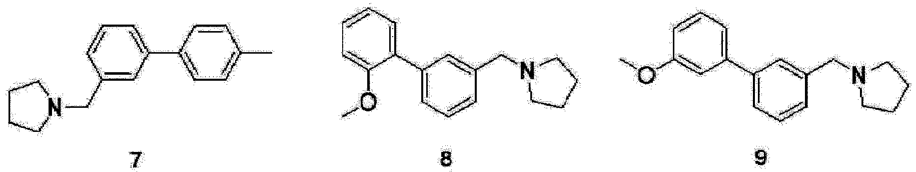

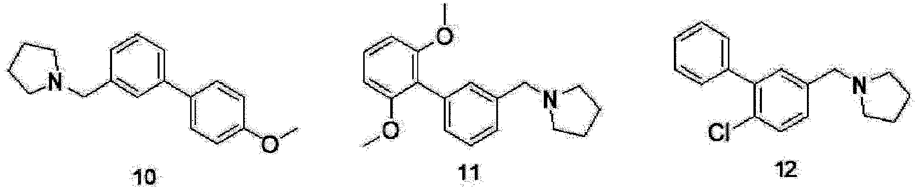

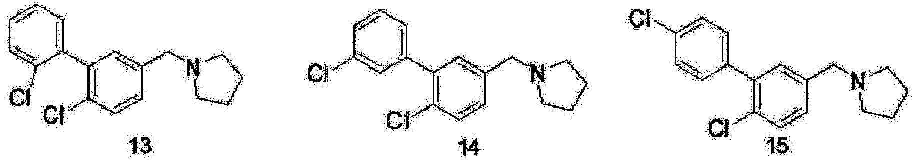

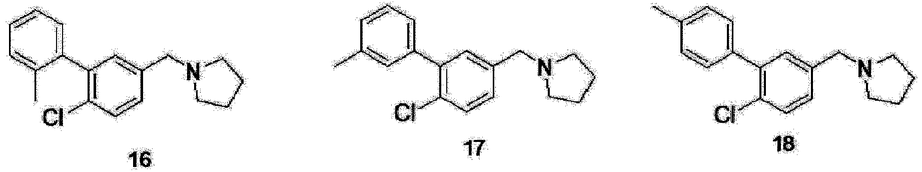

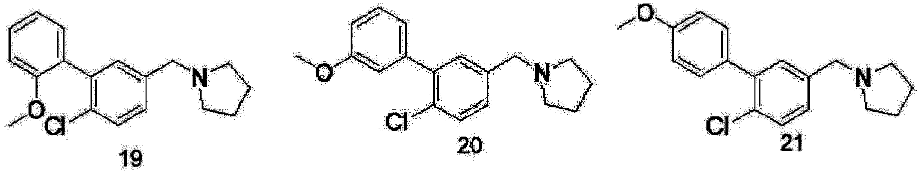

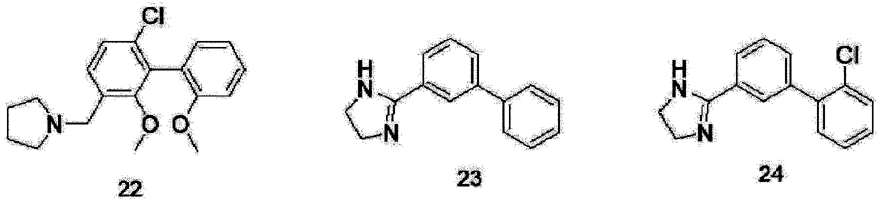

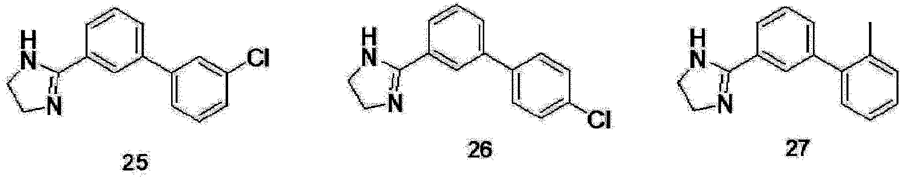

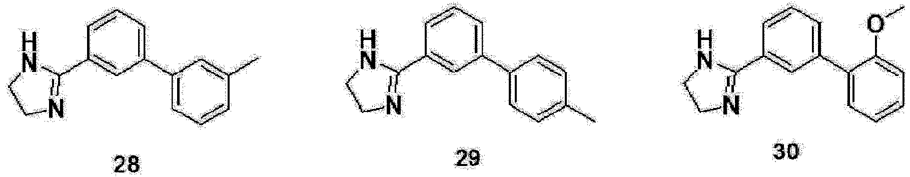

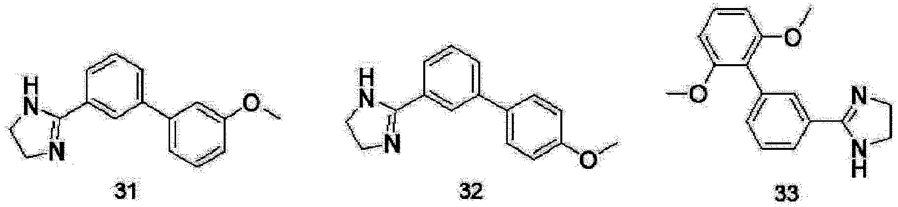

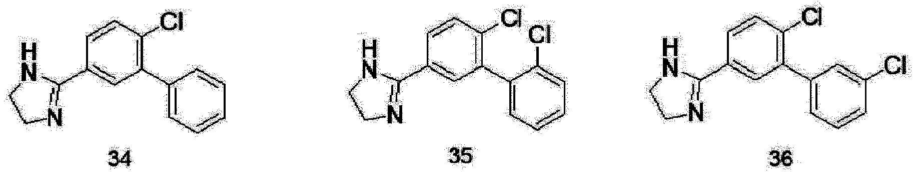

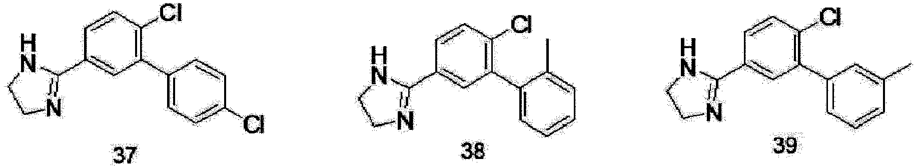

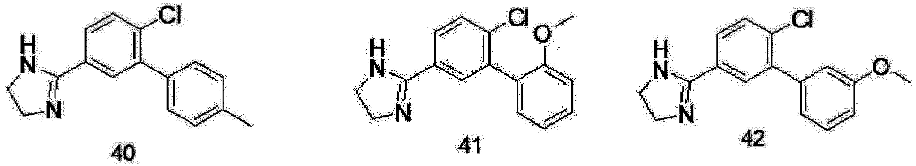

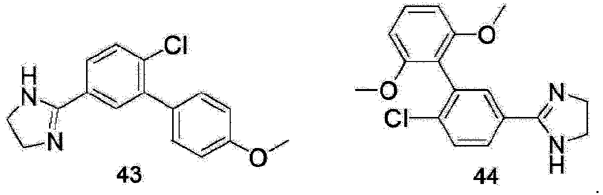

5. The compound for inhibiting the activity of the 5-HT₇ serotonin receptor or a pharmaceutically acceptable salt thereof according to claim 1, wherein the pharmaceutically acceptable salt is a salt formed using any inorganic acid or organic acid selected from hydrochloric acid, bromic acid, sulfonic acid, amidosulfuric acid, phosphoric acid, nitric acid, acetic acid, propionic acid, succinic acid, glycolic acid, stearic acid, lactic acid, tartaric acid, citric acid, p-toluenesulfonic acid and methanesulfonic acid.

6. A method for preparing a compound represented by Structural Formula 1 or 2 for inhibiting the activity of the 5-HT₇ serotonin receptor, comprising a step of reacting a compound represented by Structural Formula A with pyrrolidine or ethylenediamine: 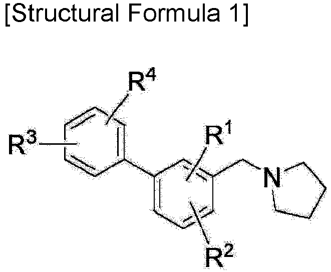 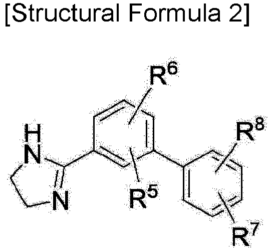 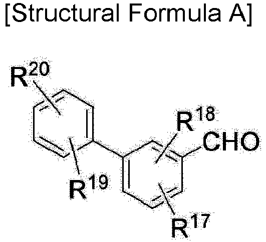 wherein,
in Structural Formula 1 or 2,
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group, and
in Structural Formula B,
each of R¹⁷ to R²⁰, which are identical to or different from each other, is independently a hydrogen atom, a halogen group, a substituted or unsubstituted C₁-C₁₀ alkyl group or a substituted or unsubstituted C₁-C₁₀ alkoxy group.

7. The method for preparing a compound for inhibiting the activity of the 5-HT₇ serotonin receptor according to claim 6, wherein the compound represented by Structural Formula 1 or 2 is represented by Structural Formula 3 or 4 and the compound represented by Structural Formula A is represented by Structural Formula B: 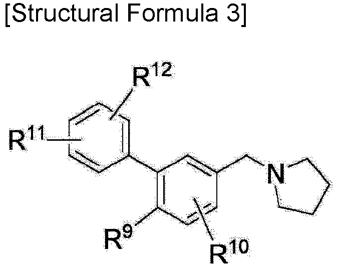 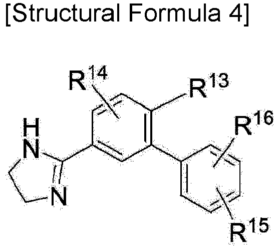 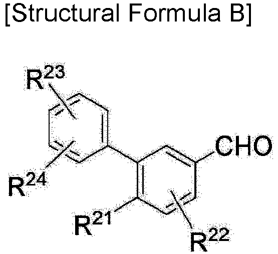 wherein,
in Structural Formula 3 or 4,
each of R⁹ to R¹⁶, which are identical to or different from each other, is independently a hydrogen atom, a halogen group, a substituted or unsubstituted C₁-C₁₀ alkyl group or a substituted or unsubstituted C₁-C₁₀ alkoxy group, and
in Structural Formula B,
each of R²¹ to R²⁴, which are identical to or different from each other, is independently a hydrogen atom, a halogen group, a substituted or unsubstituted C₁-C₁₀ alkyl group or a substituted or unsubstituted C₁-C₁₀ alkoxy group.

8. The method for preparing a compound for inhibiting the activity of the 5-HT₇ serotonin receptor according to claim 7, wherein the compound represented by Structural Formula B is prepared by the Suzuki reaction of a compound represented by Structural Formula C and a compound represented by Structural Formula D: 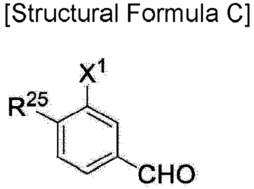 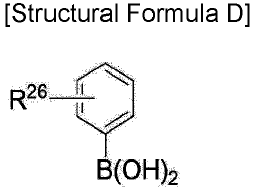 wherein
X₁ is a bromo group or an iodo group, and
each of R²⁵ and R²⁶, which are identical to or different from each other, is independently a hydrogen atom, a halogen group, a substituted or unsubstituted C₁-C₁₀ alkyl group or a substituted or unsubstituted C₁-C₁₀ alkoxy group.

9. The method for preparing a compound for inhibiting the activity of the 5-HT₇ serotonin receptor according to claim 8, wherein R²⁵ is a hydrogen atom and X₁ is a bromo group.

10. The method for preparing a compound for inhibiting the activity of the 5-HT₇ serotonin receptor according to claim 8, wherein R²⁶ is a chloro group and X₁ is an iodo group.

11. A pharmaceutical composition for preventing or treating a central nervous system disease, comprising a compound represented by Structural Formula 1 or 2 or a pharmaceutically acceptable salt thereof as an active ingredient: 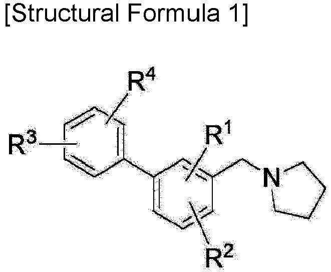 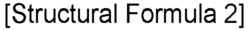 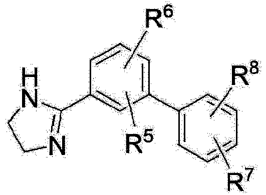 wherein
each of R¹ to R⁸, which are identical to or different from each other, is independently a hydrogen atom, a halogen group, a substituted or unsubstituted C₁-C₁₀ alkyl group or a substituted or unsubstituted C₁-C₁₀ alkoxy group.

12. The pharmaceutical composition for preventing or treating a central nervous system disease according to claim 11, wherein the compound represented by Structural Formula 1 or 2 is any one selected from the following compounds 1-44:

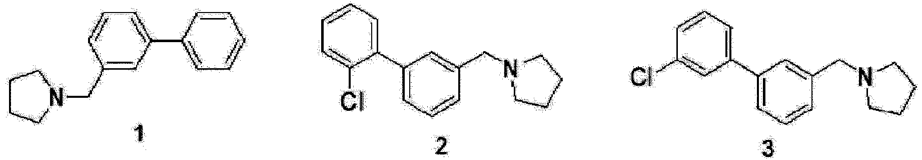

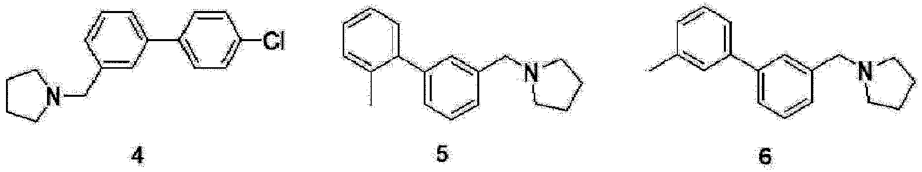

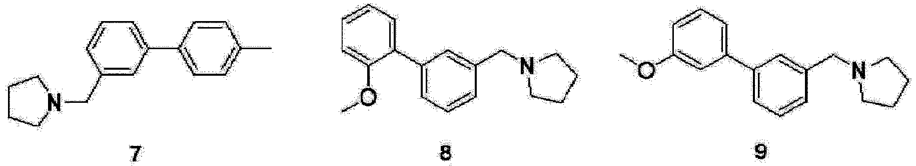

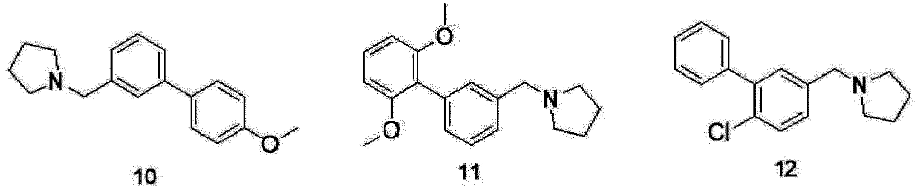

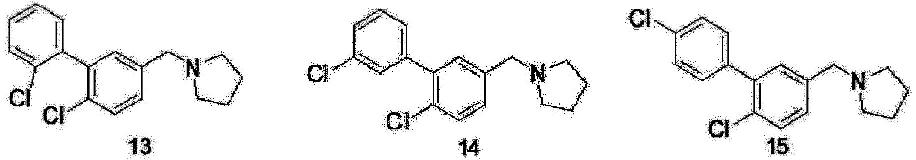

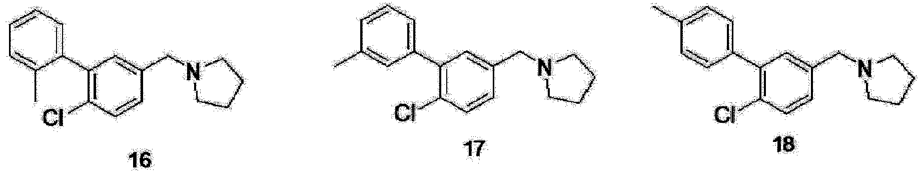

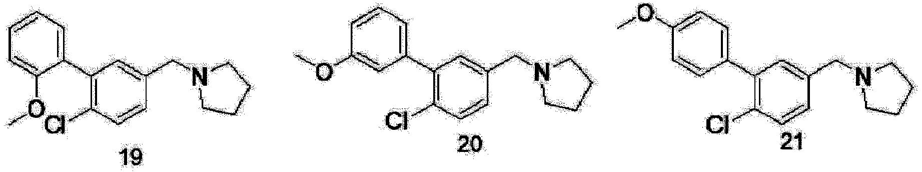

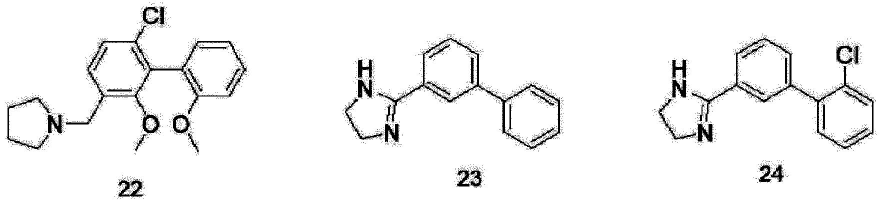

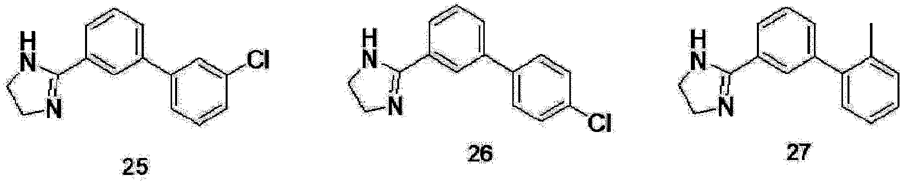

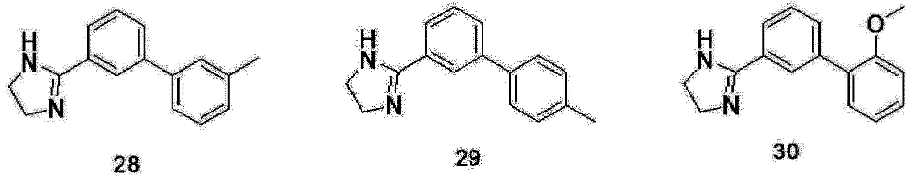

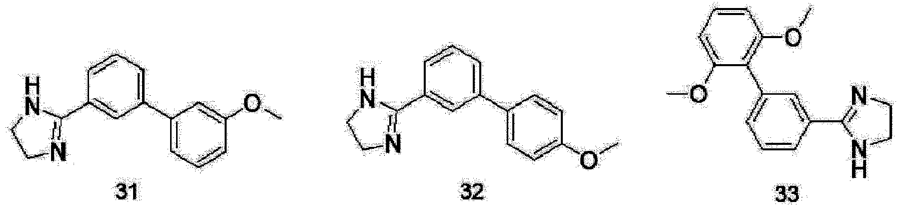

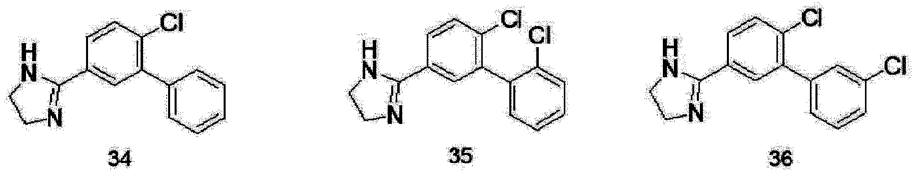

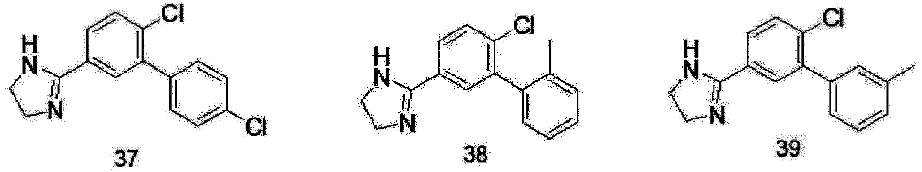

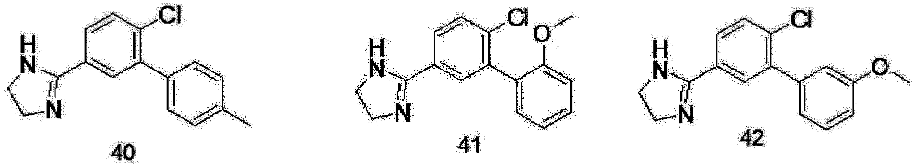

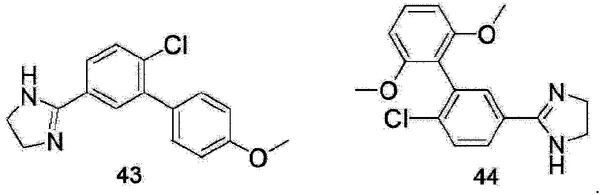

13. The pharmaceutical composition for preventing or treating a central nervous system disease according to claim 11, wherein the central nervous system disease is any disease selected from sleep disorder, depression, migraine, anxiety, pain, inflammatory pain, neuropathic pain, thermoregulation disorder, biorhythm disorder, autism spectrum disorder and smooth muscle disorder.
